(19) **Europäisches Patentamt**
**European Patent Office**
**Office européen des brevets**

(11) **EP 2 910 279 B1**

(12) **EUROPEAN PATENT SPECIFICATION**

(45) Date of publication and mention
of the grant of the patent:
**04.07.2018 Bulletin 2018/27**

(51) Int Cl.:
***A61N 5/10*** *(2006.01)*

(21) Application number: **15156580.1**

(22) Date of filing: **25.02.2015**

(54) **BEAM POSITION MONITORING APPARATUS AND CHARGED PARTICLE BEAM IRRADIATION SYSTEM**

STRAHLPOSITIONSÜBERWACHUNGSVORRICHTUNG UND SYSTEM ZUR BESTRAHLUNG MIT GELADENEN TEILCHEN

APPAREIL DE SURVEILLANCE DE POSITION DE FAISCEAU ET SYSTÈME D'IRRADIATION À FAISCEAU DE PARTICULES CHARGÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.02.2014 JP 2014034015**

(43) Date of publication of application:
**26.08.2015 Bulletin 2015/35**

(73) Proprietor: **Hitachi, Ltd.**
**Tokyo 100-8280 (JP)**

(72) Inventors:
 • **Nishimura, Arao**
  **Tokyo, 100-8280 (JP)**
 • **Akiyama, Hiroshi**
  **Tokyo, 100-8280 (JP)**
 • **Shinagawa, Ryosuke**
  **Tokyo, 100-8280 (JP)**
 • **Fujita, Takeshi**
  **Tokyo, 100-8280 (JP)**

(74) Representative: **MERH-IP Matias Erny Reichl Hoffmann**
**Patentanwälte PartG mbB**
**Paul-Heyse-Strasse 29**
**80336 München (DE)**

(56) References cited:
 **JP-A- 2009 039 219   US-A1- 2012 161 030**
 **US-A1- 2013 026 388   US-B1- 6 600 164**
 **US-B1- 6 677 597**

 • **REIMOSER S A ET AL: "Engineering design and study of the beam position accuracy in the "Riesenrad" ion gantry", NUCLEAR INSTRUMENTS & METHODS IN PHYSICS RESEARCH. SECTION A: ACCELERATORS, SPECTROMETERS, DETECTORS, AND ASSOCIATED EQUIPMENT, ELSEVIER BV * NORTH-HOLLAND, NL, vol. 456, no. 3, 1 January 2001 (2001-01-01), pages 390-410, XP004226991, ISSN: 0168-9002, DOI: 10.1016/S0168-9002(00)00577-5**

**Description**

BACKGROUND OF THE INVENTION

[Technical Field]

The present invention relates to a beam position monitoring apparatus and a charged particle beam irradiation system and more particularly, to a beam position monitoring apparatus and a charged particle beam irradiation system that are suitable for monitoring the irradiation position of an ion beam such as a proton or a carbon ion to a tumor volume.

[Background Art]

A method of irradiating an ion beam such as a proton and carbon to the tumor volume of a patient by using the charged particle beam irradiation system and thereby treating the cancer is known. The charged particle beam irradiation system includes an ion source, an accelerator, a beam transport system, a rotating gantry, and an irradiation nozzle. A synchrotron and a cyclotron are known as an accelerator to be used in the charged particle beam irradiation system.

An ion beam generated in the ion source is accelerated up to desired energy using the accelerator such as the synchrotron or cyclotron and then is extracted from the accelerator to the beam transport system. The extracted ion beam is transported to the irradiation nozzle installed on the rotating gantry by the beam transport system. The rotating gantry is rotated, thus the irradiation nozzle is rotated around a rotary shaft and is fitted to an ion beam irradiation direction to the tumor volume of the patient lying on a treatment bed. Therefore, the ion beam transported to the irradiation nozzle is applied in correspondence to depth of a target volume, which is an irradiation target of the ion beam, from a body surface and shape of the target volume in the irradiation direction set by the rotating gantry.

There are a scatterer method and a beam scanning method as a main irradiation method of the ion beam to the tumor volume. In the scatterer method, the ion beam is enlarged in a direction perpendicular to an axial center of the irradiation nozzle by using a scatterer and the ion beam formed in correspondence to the sectional shape of the target volume in the direction perpendicular to the axial center thereof by using a collimator is applied to the target volume. In the beam scanning method, the ion beam is scanned in the direction perpendicular to the axial center of the irradiation nozzle in correspondence to the target volume shape by using a scanning magnet, and the ion beam energy is changed by the accelerator or a degrader, and the target volume is irradiated with the ion beam in the depth direction.

The ion beam irradiation to the target volume by the scatterer method and the beam scanning method is determined by the ion beam irradiation mechanism installed on the irradiation nozzle. In the charged particle beam irradiation system to which the scatterer method is applied, a scatterer, a ridge filter, and a collimator are installed in the irradiation nozzle as the ion beam irradiation mechanism. In the charged particle beam irradiation system to which the beam scanning method is applied, the scanning magnet for scanning the ion beam is installed on the irradiation nozzle as the ion beam irradiation mechanism. The ion beam accelerated in the accelerator can be effectively used to the irradiation to the target volume by using the beam scanning method.

An example of the charged particle beam irradiation system using the beam scanning method is described in Japanese Patent Laid-open No. 10(1998)-118204, Japanese Patent Laid-open No. 2004-358237, and Japanese Patent Laid-open No. 2011-177374. These publications describe the charged particle beam irradiation method of dividing a tumor volume into a plurality of layers from a body surface in the ion beam irradiation direction, scanning a narrow ion beam, and thereby irradiating a plurality of irradiation spots which are the irradiation positions in each layer with the ion beam. The movement of the ion beam to the neighboring irradiation spot in each layer is executed by controlling the scanning magnet of changing the ion beam position by a scanning control apparatus. Further, the movement of the ion beam from a distal layer to a proximal layer (or from the proximal layer to the distal layer) is executed by changing the energy of the ion beam by the accelerator or the degrader. As the energy of the ion beam increases, the bragg peak described later of the ion beam reaches a distal position of the human body.

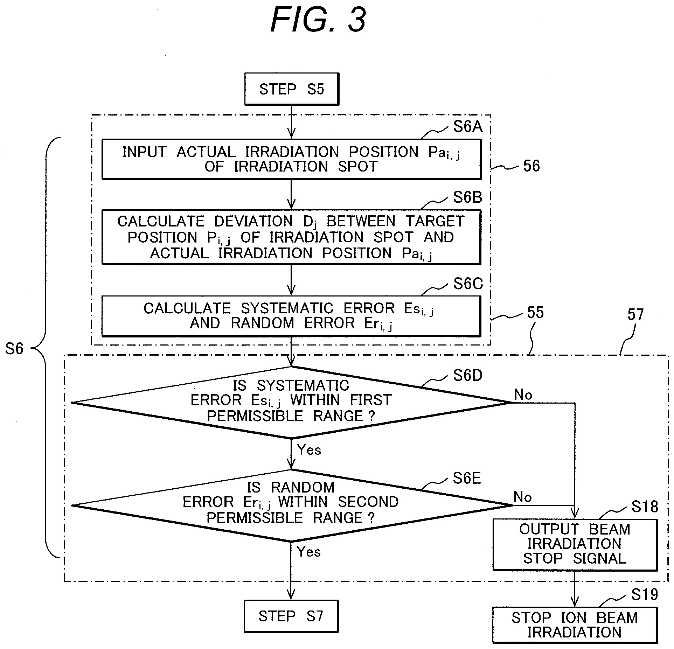

Even in the beam scanning method, when the human body is irradiated with the ion beam, the dose distribution as shown in FIG. 3 of Japanese Patent Laid-open No. 10(1998)-118204 is shown in the depth direction of the human body. The dose is maximized at the bragg peak, and furthermore, the dose distribution reduces suddenly at the depth exceeding the bragg peak. The cancer treatment using the ion beam uses the property that the dose is maximized at the bragg peak and the dose is suddenly reduced at a depth exceeding the bragg peak.

In the beam scanning method, when irradiating the ion beam to each target position of the respective irradiation spots in each layer, the ion beam must be irradiated within the permissible range corresponding to the target position of each irradiation spot to suppress the dose distribution uniformity within the permissible range. If outside of the corresponding permissible range is irradiated with the ion beam, the ion beam irradiation to the target volume is stopped (Japanese Patent Laid-open No. 2011-177374 and Japanese Patent Laid-open No. 2011-206495).

[Citation List]

[Patent Literature]

[Patent Literature 1] Japanese Patent Laid-open No. 10 (1998)-118204
[Patent Literature 2] Japanese Patent Laid-open No. 2004-358237
[Patent Literature 3] Japanese Patent Laid-open No. 2011-177374
[Patent Literature 4] Japanese Patent Laid-open No. 2011-206495
[Patent Literature 5] US 2012/161030 A1 describes a particle beam irradiation apparatus. There is provided a data processing apparatus that displays on a display unit a measured irradiation position value and an irradiation position value error, which is the error of the measured irradiation position relevant value related to the irradiation position of charged particle beam with respect to a desired irradiation position value related to a desired irradiation position.

SUMMARY OF THE INVENTION

[Technical Problem]

Size of the irradiation spots is narrowed in diameter, so that the concentration of the ion beam to the target volume can be enhanced. However, when the ion beam is narrowed in diameter, the sensitivity of the uniformity of the dose distribution to an error in the irradiation position of the ion beam is enhanced, so that higher position accuracy is required for the irradiation spots which are irradiated with the ion beam. Therefore, the monitoring of the uniformity of the dose distribution to the target volume must be made severe. To make the monitoring of the uniformity of the dose distribution severe leads to narrowing the permissible range of the irradiation spots for the error between the target position of the irradiation spot and the actual irradiation position. However, if the permissible range for the irradiation spots is narrowed, the entire dose distribution of the target volume is not affected. Even when an error in an actual irradiation spot position is caused, the irradiation spot deviates from the permissible range. Under the circumstances, the cancer treatment cannot be executed stably.

When irradiating the ion beam to the irradiation spot by the beam scanning method, the derivation of the irradiated ion beam from the permissible range of the irradiation spot is caused based on an error between the target position of the irradiation spot set by the treatment planning and the actual irradiation position of the irradiation spot which was irradiated with the ion beam. In the charged particle beam irradiation method described in Japanese Patent Laid-open No. 2011-177374 and Japanese Patent Laid-open No. 2011-206495, the error is large and when the position of the irradiation spot which was irradiated with the ion beam is deviated from the permissible range for the target position of the irradiation spot, the ion beam irradiation to the target volume is stopped.

When the irradiation stop of the ion beam to the target volume is caused, the charged particle beam irradiation system must be inspected to find the cause of the deviation of the error between the target position of the irradiation spot set by the treatment planning and the actual irradiation position of the irradiation spot which was irradiated with the ion beam from the permissible range. If a defective portion for generating such an error exists in the charged particle beam irradiation system, the defective portion must be repaired. A treatment planning not causing such an error must be prepared. The inspection and repair of the charged particle beam irradiation system may require a long period of time.

Therefore, when the error between the target position of the irradiation spot and the actual irradiation position of the irradiation spot is deviated from the permissible range and the ion beam irradiation to the target volume is stopped, the number of persons that can be treated per day is reduced.

An object of the present invention is to provide a beam position monitoring apparatus and a charged particle beam irradiation system that are capable of reducing an unscheduled stop of ion beam irradiation and increasing the number of persons capable of being treated per day.

[Solution to Problem]

The above object is attained by the invention as set forth in the independent claims. Preferred developments are described by the dependent claims.

The deviation between the target position and the actual irradiation position is obtained, and the systematic error and random error are obtained based on this deviation as an error of the actual irradiation position of the charged particle beam against the target position which is irradiated with the charged particle beam, and whether the systematic error exists within the first permissible range of the systematic error and whether the random error exists within the second permissible range of the random error are determined separately, so that probability that the systematic error deviates from the first permissible range and furthermore, the random error deviates from the second permissible range is reduced. Therefore, probability of irradiation stop of the charged particle beam to the beam irradiation subject, for example, the target volume, is reduced remarkably and an unscheduled stop of the irradiation with the charged particle beam to the beam irradiation subject using the charged particle beam irradiation system is reduced extremely. As a result, the number of persons that can be treated per day can be increased.

[Advantageous Effect of the Invention]

According to the present invention, an unscheduled stop of the ion beam irradiation can be reduced and the number of persons that can be treated per day can be increased.

BRIEF DESCRIPTION OF THE DRAWINGS

FIG. 1 is a structural diagram showing a charged particle beam irradiation system according to embodiment 1 which is a preferred embodiment of the present invention.
FIG. 2A is a flow chart showing a part of a procedure of a method of irradiating charged particle beam using the charged particle beam irradiation system shown in FIG. 1.
FIG. 2B is a flow chart showing a remaining portion of a procedure of a method of irradiating charged particle beam using a charged particle beam irradiation system shown in FIG. 1.
FIG. 3 is a flow chart showing a detailed procedure at step S6 shown in FIG. 2A.
FIG. 4 is an explanatory drawing showing respective permissible ranges of systematic error and random error.
FIG. 5 is an explanatory drawing showing region division (layer division) in a depth direction from a body surface of tumor volume of a patient receiving treatment by irradiation with ion beam.
FIG. 6 is an explanatory drawing showing an example of a dose distribution irradiated in each layer to obtain a uniform dose distribution in a depth direction of a target region (target volume) which was irradiated with ion beam.
FIG. 7 is an explanatory drawing showing an example of a shift between an irradiation spot position of a target in a certain layer of target volume and the irradiation spot position which was actually irradiated with ion beam.
FIG. 8 is an explanatory drawing showing variations of an actual irradiation position of an irradiation spot which was irradiated with ion beam when a target position of an irradiation spot was irradiated with ion beam.
FIG. 9 is an explanatory drawing showing a conventional permissible range for irradiation spot position of target.
FIG. 10 is an explanatory drawing showing respective permissible ranges of a systematic error and a random error included in a error between an irradiation spot position of target and an irradiation spot position which was actually irradiated with ion beam.
FIG. 11 is a structural diagram showing a charged particle beam irradiation system according to an example being useful for the understanding of the present invention.
FIG. 12 is a flow chart showing a part of a procedure of a method of irradiating charged particle beam using a charged particle beam irradiation system shown in FIG. 11.
FIG. 13 is an explanatory drawing showing the timing of executing respective determinations of a systematic error and a random error in a spot and a beam irradiation section in the spot when a procedure shown in FIG. 12 is executed.
FIG. 14 is a structural diagram showing a charged particle beam irradiation system according to embodiment 2 which is other preferred embodiment of the present invention.

DETAILED DESCRIPTION OF THE PREFERRED EMBODIMENTS

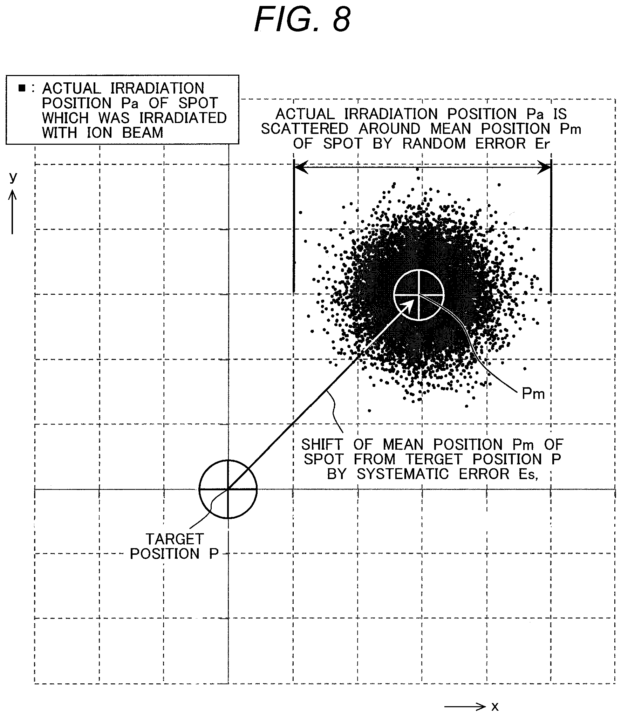

The inventors investigated a measure of reducing an unscheduled stop caused by an error between a target position of an irradiation spot set by treatment planning and an actual irradiation position of an irradiation spot which was irradiated with ion beam.
As described in Japanese Patent Laid-open No. 2011-177374 and Japanese Patent Laid-open No. 2011-206495, a phenomenon that the irradiation spot which is irradiated with the ion beam deviates from a corresponding permissible range and that it is stopped that target volume is irradiated with the ion beam is generated by an increase in size of an error between a target position of the irradiation spot and an actual irradiation position of the irradiation spot which is irradiated with the ion beam. Therefore, the inventors focused attention on the error between the target position of the irradiation spot and the actual irradiation position of the irradiation spot which is irradiated with the ion beam.
The actual irradiation position of each irradiation spot which is irradiated with the ion beam when the target position of the irradiation spot is irradiated with the ion beam is shown in FIG. 8. In FIG. 8, the actual irradiation position of each irradiation spot which was irradiated with the ion beam is shown by a small square point. In the example shown in FIG. 8, the actual irradiation position of the irradiation spot is shifted in the upper right for a target position P of the irradiation spot and the actual irradiation position of each irradiation spot is also scattered around a mean position (a center of gravity) Pm of the actual irradiation positions of these irradiation spots.

A systematic error that generates a fixed amount of position shift from the target position of the irradiation spot and a random error that generates a shift from the mean position Pm of the actual irradiation position of the irradiation spot exist as an error of the actual irradiation position of the irradiation spot which was irradiated with the ion beam.

The systematic error is a shift of the mean position Pm of the actual irradiation position of the irradiation spot which was irradiated with the ion beam from the target position P of the irradiation spot. In other words, the mean position Pm of the actual irradiation position is shifted from the target position of the irradiation spot because of the systematic error. This systematic error is generated caused by an inclination angle of a rotating gantry of a charged particle beam irradiation system and a bending magnet attached to the rotating gantry.

To obtain a systematic error $Es_j$ ($j = 1, 2, ..., n$), firstly, a deviation $D_j$ between a target position $Pj$ ($j = 1, 2, ..., n$) of the irradiation spot which was irradiated with the ion beam and an actual irradiation position $Pa_j$ ($j = 1, 2, ..., n$) of the irradiation spot which was irradiated with the ion beam is calculated from formula (1).

$$ D_j = P_j - P a_j \qquad \cdots (1) $$

The actual irradiation position $Pa_j$ is one for the j-th ($j = 1, 2, ..., n$) irradiation spot in a certain layer.

The systematic error $Es_j$, concretely, is a shift of the mean position $Pm_j$ ($j = 1, 2, ..., n$) of the j ($j = 1, 2, ..., n$) actual irradiation positions $Pa_j$ from the target position $P_j$ ($j = 1, 2, ..., n$) of the irradiation spot. The systematic error $Es_j$ ($j = 1, 2, ..., n$) is obtained both in the x direction and in the y direction.

The random error indicates a shift of the actual irradiation position of each irradiation spot from the mean position Pm of these actual irradiation positions. If the random error becomes larger, the uniformity of the dose distribution of the target volume is deteriorated. The random error is generated by stability of a synchrotron, stability of a transport line magnet power supply for transporting the beam from the synchrotron to an irradiation nozzle, a measurement error of a beam position monitor installed on the irradiation nozzle, and the scanning of the ion beam by a scanning magnet.

The random error $Er_j$ ($j = 1, 2, ..., n$) is obtained both in the x direction and in the y direction.

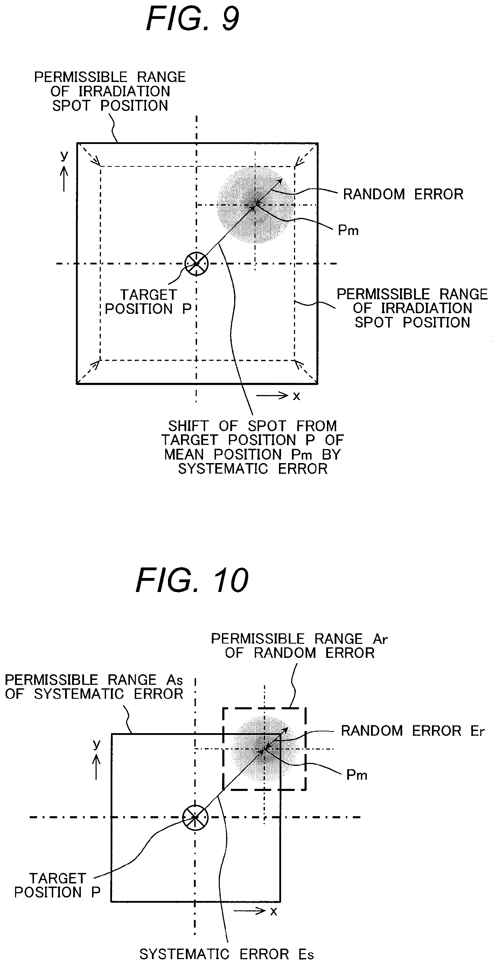

As described in Japanese Patent Laid-open No. 2011-177374 and Japanese Patent Laid-open No. 2011-206495, conventionally, one permissible range is set for the target position of the irradiation spot. Namely, as shown in FIG. 9, one permissible range of the irradiation spot position is set for a target position P of the irradiation spot. The permissible range covers the aforementioned systematic error and random error.

However, the irradiation spot which is irradiated with the ion beam is thinned in diameter, thus the sensitivity of the dose distribution to an error of the irradiation spot position is enhanced. Thus, the monitoring of the uniformity of the dose distribution must be made severe.

To realize severe monitoring of an error in correspondence with the diameter thinning of the irradiation spot, as shown in FIG. 9, the permissible range shown by a solid line of the irradiation spot position must be narrowed within the permissible range shown by a dashed line of the irradiation spot position. If the permissible range of the irradiation spot position is narrowed as shown by the dashed line, when the target volume is irradiated with the ion beam, a case that the actual irradiation position of the irradiation spot deviates from the permissible range of the irradiation spot position shown by the dashed line increases, and frequency of an unscheduled stop of the ion beam irradiation to the target volume increases. Under the circumstances, the charged particle beam irradiation system cannot be operated stably. A stable operation of the charged particle beam irradiation system is desired to increase the number of persons capable of being treated per day.

To respond to such a need, the inventors thought of setting separately the permissible range for the respective systematic error and random error and separately monitoring the systematic error and random error based on each permissible range.

Referring to FIG. 10, the permissible range for the systematic error and the permissible range for the random error will be explained.

The shift of the mean position Pm of the actual irradiation position of each irradiation spot which was irradiated with the ion beam from the target position P of the irradiation spot is a systematic error Es. For the systematic error Es, a permissible range As (a first permissible range) of the systematic error based on the target position P of the irradiation spot is set. The permissible range As includes upper limit values (+Asx, +Asy) and lower limit values (-Asx, -Asy), respectively, based on the target position P in the x direction and the y direction perpendicular to it.

The shift of the actual irradiation position of the irradiation spot which was irradiated with the ion beam from the mean position Pm of the actual irradiation position of each irradiation spot is a random error Er. For the random error Er, a permissible range Ar (a second permissible range) of the random error based on the mean position Pm of the actual irradiation position is set. The permissible range Ar also includes upper limit values (+Arx, +Ary) and lower limit values (-Arx, -Ary), respectively, based on the mean position Pm of the actual irradiation position in the X direction and the Y direction perpendicular to it.

The permissible range As of the systematic error is narrower than the permissible range of the conventional spot position shown in FIG. 9. Further, the permissible range Ar of the random error is narrower than the permissible range As of the systematic error. For the systematic error Es and random error Er, as mentioned above, the permissible range As and the permissible range Ar are respectively set, so that the systematic error Es and random error Er can be monitored severely.

As mentioned above, even when severe monitoring is executed for both the systematic error and random error, the permissible range of the systematic error and the permissible range of the random error are set separately, so that the probability that the systematic error deviates from the permissible range of the systematic error and furthermore the random error deviates from the permissible range of the random error is reduced. Therefore, the probability of irradiation stop of the ion beam to a patient is reduced extremely, and a stabler operation of the charged particle beam irradiation system is enabled, and the unscheduled stop of the ion beam irradiation to the patient is reduced extremely. As a result, the number of persons capable of being treated per day can be increased.

The embodiments of the present invention with the above investigation results reflected will be explained below.

[Embodiment 1]

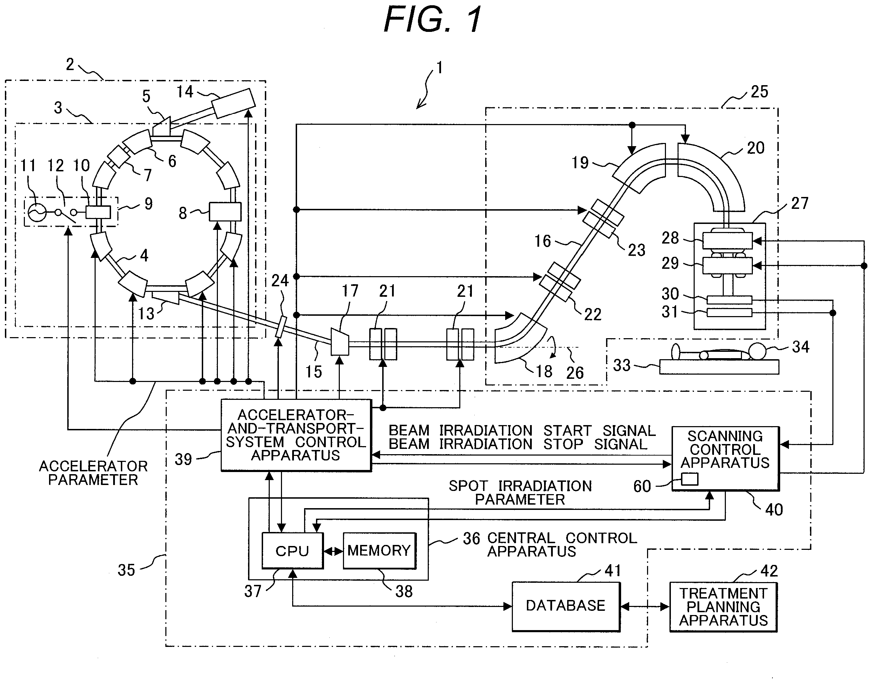

A charged particle beam irradiation system according to embodiment 1 which is a preferred embodiment of the present invention will be explained by referring to FIG. 1. In a charged particle beam irradiation system 1 of the present embodiment, a proton ion beam is used as an ion beam applied to the tumor volume which is an irradiation target. Instead of the proton ion beam, a carbon ion beam may be used.

The charged particle beam irradiation system 1 of the present embodiment is provided with a charged particle generating apparatus 2, a beam transport system 15, a rotating gantry 25, an irradiation nozzle 27, and a control system 35. The charged particle generating apparatus 2 uses a synchrotron accelerator 3 as an accelerator and as shown in FIG. 1 includes a linear accelerator 14 which is a preceding accelerator other than the synchrotron accelerator 3.

The synchrotron accelerator 3 includes a circular beam duct 4 configuring a circular orbit of the ion beam, an injector 5, an acceleration apparatus (an acceleration cavity) 8 of applying a radiofrequency voltage to the ion beam, a plurality of bending magnets 6, a plurality of quadrupole magnets 7, a radiofrequency application apparatus 9 for extraction, and an extraction deflector 13. The injector 5 connected with the beam duct 4 is connected to the linear accelerator 14 by the vacuum duct which is a beam path. The radiofrequency application apparatus 9 includes an extraction radiofrequency electrode 10, a radiofrequency power supply 11, and an open/close switch 12. The extraction radiofrequency electrode 10 is installed in the beam duct 4 and is connected to the radiofrequency power supply 11 through the open/close switch 12. The acceleration apparatus 8, each bending magnet 6, each quadrupole magnet 7, and the extraction deflector 13 are disposed along the beam duct 4, as shown in FIG. 1. The radiofrequency power supply apparatus (not shown) is connected to the acceleration apparatus 8.

The beam transport system 15 includes a beam path (beam duct) 16 reaching the irradiation nozzle 27 and the beam path 16 is structured so that a bending magnet 17, a plurality of quadrupole magnets 21, a bending magnet 18, quadrupole magnets 22 and 23, and bending magnets 19 and 20 are disposed in this order from the synchrotron accelerator 3 toward the irradiation nozzle 27. A shutter 24 made of a radiation shielding material is attached to the beam path 16 so as to be opened or closed between the extraction deflector 13 and the bending magnet 17. A part of the beam path 16 of the beam transport system 15 is installed on the rotating gantry 25. The bending magnet 18, the quadrupole magnets 22 and 23, and the bending magnets 19 and 20 are also installed on the rotating gantry 25. The beam path 16 is connected to the circular beam duct 4 of the synchrotron accelerator 3 in the neighborhood of the extraction deflector 13. The rotating gantry 25 is structured so as to rotate around a rotary shaft 26.

The irradiation nozzle 27 includes two scanning magnets (charged particle beam scanning apparatuses) 28 and 29, a beam position monitor 30, and a dose monitor 31. The scanning magnets 28 and 29, the beam position monitor 30, and the dose monitor 31 are disposed along a central axis of the irradiation nozzle 27. The scanning magnets 28 and 29, the beam position monitor 30, and the dose monitor 31 are disposed in a casing (not shown) of the irradiation nozzle 27 and the beam position monitor 30 and the dose monitor 31 are disposed on the downstream side of the scanning magnets 28 and 29. The scanning magnet 28 bends the ion beam in a plane perpendicular to the central axis of the irradiation nozzle 27 and scans it in a y direction. The scanning magnet 29 bends the ion beam in the plane and scans it in a x direction perpendicular to the y direction. The irradiation nozzle 27 is attached to the rotating gantry 25 and is disposed on the downstream side of the bending magnet 20. A treatment bed 33 with a patient 34 lying on is disposed so as to be opposite to the irradiation nozzle 27.

The control system 35 includes a central control apparatus 36, an accelerator-and-transport-system control apparatus 39, a scanning control apparatus 40, and a data base 41. The central control apparatus 36 includes a central processing unit (CPU) 37 and a memory 38 connected to the CPU 37. The accelerator-and-transport-system control apparatus 39, the scanning control apparatus 40, and the data base 41 are connected to the central processing unit 37. The charged particle beam irradiation system 1 includes a treatment planning apparatus 42 and the treatment planning apparatus 42 is connected to the data base 41.

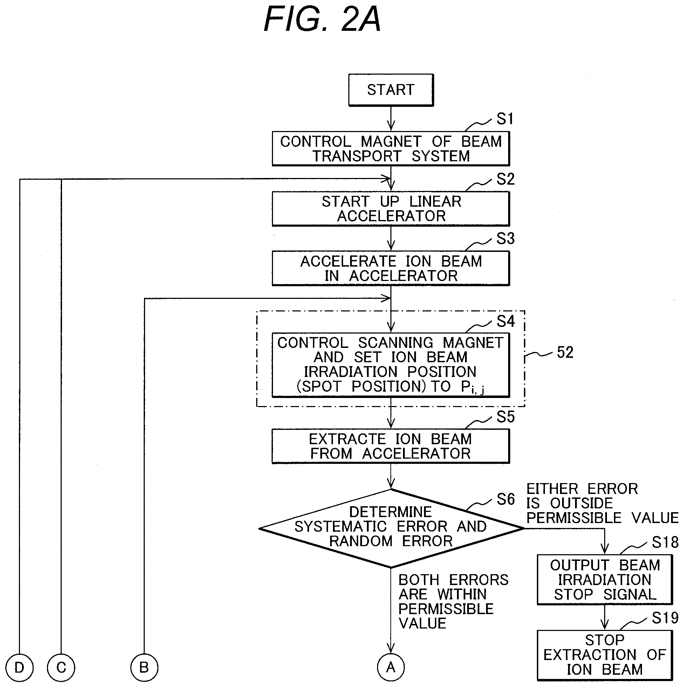

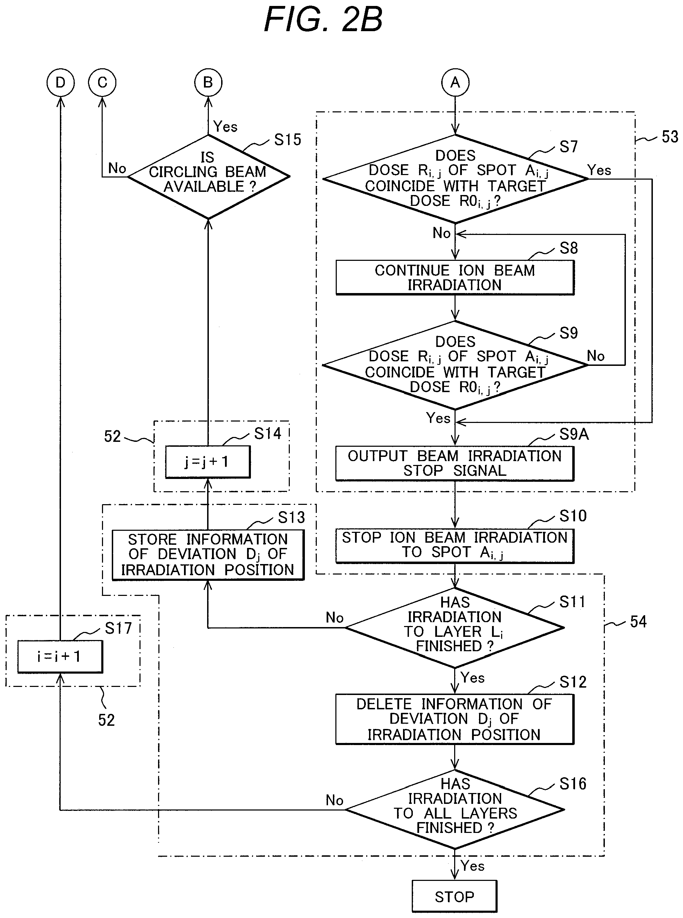

The scanning control apparatus 40 includes an irradiation position control apparatus 52, a dose determination apparatus (a first dose determination apparatus) 53, a layer determination apparatus 54, and a beam position monitoring apparatus 55, as shown in FIGs. 2A, 2B, and 3. The beam position monitoring apparatus 55 includes an error operating apparatus (a first error operating apparatus) 56 and an error determination apparatus (a first error determination apparatus) 57.

A method of irradiating charged particle beam using the charged particle beam irradiation system 1 will be explained below.

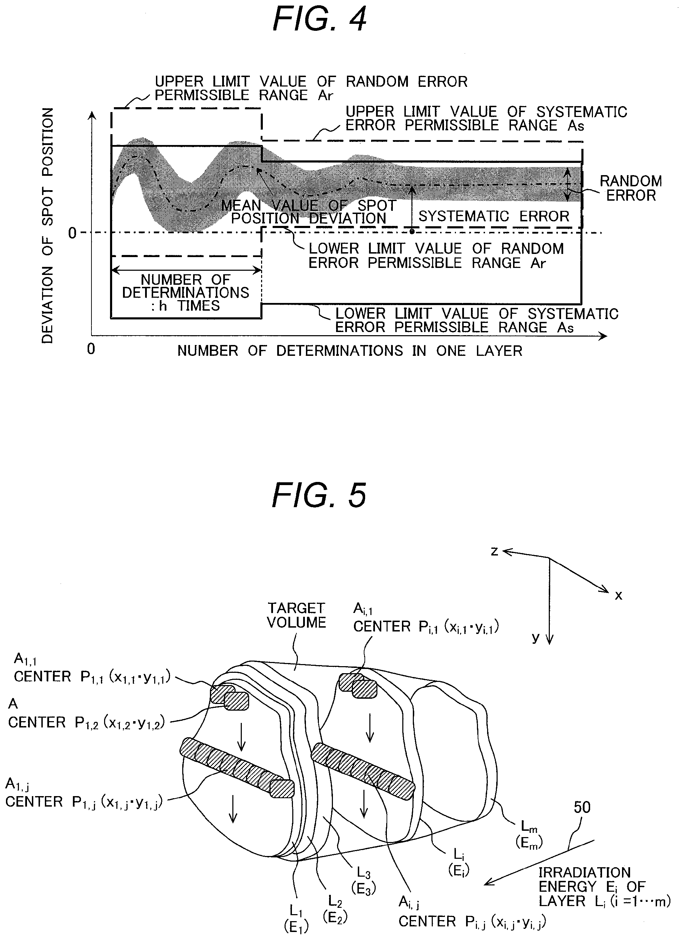

The treatment planning for the target volume of a patient treated using the charged particle beam irradiation system 1 is executed using the treatment planning apparatus 42. The outline of the treatment planning will be explained below. The position and shape of the tumor volume are recognized by using tomographic image information of the patient photographed by an X-ray CT apparatus. An irradiation direction of a proton ion beam (hereinafter simply referred to as an ion beam) for the target volume is determined and the target volume is divided into a plurality of layers $L_i$ (i = 1, 2, ..., m), that is, layers $L_1$, $L_2$, $L_3$, ..., and $L_m$ (refer to FIG. 5) in the irradiation direction (a depth direction from a body surface of the patient). The layer $L_1$ exists in the deepest position from the body surface. The layer depth becomes shallow in the order of the layers $L_2$, $L_3$, ..., and $L_m$, and the layer $L_m$ is most shallowest. The ion beam is applied in the direction of an arrow 50. Furthermore, the central position (target position) Pi,j of a plurality of irradiation spots Ai,j (i = 1, 2, ..., m, j = 1, 2, ...,n) which are irradiation regions and the coordinates $(x_{i,j}, y_{i,j})$ of the central position of these spots are determined in each layer and the irradiation order of the ion beam to the irradiation spots Ai,j is determined. A target dose $R0_{i,j}$ for each irradiation spot $A_{i,j}$ is determined based on the irradiation dose necessary for the entire target volume. Energy $E_i$ of the ion beam suitable for irradiation according to the depth of each layer is determined so that the ion beam reaches each layer $L_i$ and a bragg peak is formed for each layer.

Treatment planning information obtained by the treatment planning such as the ion beam irradiation direction, the respective numbers of the layer $L_i$ and the irradiation spot $A_{i,j}$, the center position $P_{i,j}$ of the irradiation spot $A_{i,j}$, the target dose $R0_{i,j}$ for each irradiation spot $A_{i,j}$, the irradiation order of the irradiation spot $A_{i,j}$, and the energy $E_i$ of the ion beam in correspondence with each layer $L_i$, is input to the data base 41 of the control system 35 from the treatment planning apparatus 42 and is registered in the data base 41 before treatment start. The CPU 37 of the central control apparatus 36 reads the treatment planning information stored in the data base 41 and stores them in the memory 38. The permissible range As of the systematic error Es and the permissible range Ar of the random error Er are stored beforehand in the memory 38.

The CPU 37 outputs above-mentioned each piece of treatment planning information stored in the memory 38, the respective currents of the scanning magnets 28 and 29 related to the entire irradiation spots $A_{i,j}$ in each layer $L_i$, and the permissible range As of the systematic error Es and the permissible range Ar of the random error Er to the scanning control apparatus 40 and store them in a memory 60 of the scanning control apparatus 40. Further, the CPU 37 transmits all acceleration parameter information of the synchrotron accelerator 13 which is stored in the memory 38 to the accelerator-and-transport-system control apparatus 39. Each piece of acceleration parameter information is stored in a memory (not shown) of the accelerator-and-transport-system control apparatus 39. The acceleration parameter information includes excitation current of each magnet of the synchrotron accelerator 13 and the beam transport system 15 determined by the energy $E_i$ of the ion beam with which each layer $L_i$ is irradiated and the radiofrequency power applied to the acceleration apparatus 8.

The patient 34 receiving treatment is taken on the treatment bed 33. Before irradiation of the ion beam to the tumor volume of the patient 34 lying on the treatment bed 33, The rotating gantry 25 is rotated around the rotary shaft 26 of the rotating gantry 25 at a predetermined angle and the central axis of the irradiation nozzle 27 is set in the irradiation direction of the ion beam prepared by the treatment planning. As a result, the central axis of the irradiation nozzle 27 is directed to the tumor volume of the patient 34 lying on the treatment bed 33.

Thereafter, the method of irradiating the charged particle beam (the ion beam) using the charged particle beam irradiation system 1 is executed and the tumor volume of the patient 34 lying on the treatment bed 33 is irradiated with the ion beam. The method of irradiating the charged particle beam will be explained using the procedure shown in FIGs. 2A and 2B. Among the processes steps S1 to S18 described in FIGs. 2A and 2B, the processes of steps S1 to S3, S5, and S19 are executed by the accelerator-and-transport-system control apparatus 39 and each of the processes of steps S4 and S6 to S18 is executed by the scanning control apparatus 40. Among the processes of step S4, S6 to S9, S9A, and S10 to S18 which are executed by the scanning control apparatus 40, the processes of steps S4, S14, and S17 are executed by the irradiation position control apparatus 52 (refer to FIG. 2A), and the processes of steps S7 to S9 and S9A are executed by the dose determination apparatus 53 (refer to FIG. 2B), and the processes of steps S11 to S13 and S16 are executed by the layer determination apparatus 54 (refer to FIG. 2B), and the processes of steps S6A to S6C are executed by the error operating apparatus 56 (refer to FIG. 3), and the processes of steps S6D, S6E, and S18 are executed by the error determination apparatus 57 (refer to FIG. 3).

Each magnet of the beam transport system 15 is controlled (step S1). When the target volume of the patient 34 is irradiated with the ion beam, though firstly, the deepest layer $L_1$ of the target volume is irradiated with the ion beam. After the respective target positions Pi,j of all the irradiation spots Ai,j in the layer $L_1$ were irradiated with the ion beam, the layers $L_2$, $L_3$, ..., and $L_m$ is successively irradiated with the ion beam toward the layers in the shallow position. The accelerator-and-transport-system control apparatus 39 excites the bending magnets 17, 19, and 20 and the quadrupole magnets 21, 22, and 23 of the beam transport system 15 at the respective excitation currents thereof which are determined by the energy $E_1$ of the ion beam with which the layer $L_1$ is irradiated. The accelerator-and-transport-system control apparatus 39 opens the shutter 24 to introduce the ion beam accelerated and extracted from the synchrotron accelerator 3 to the irradiation nozzle 27.

The linear accelerator is started up (step S2). The accelerator-and-transport-system control apparatus 39 starts up the linear accelerator 14 and the ion source (not shown) connected to the linear accelerator 14. The ions (for example, proton ions) generated by the ion source are accelerated by the linear accelerator 14.

The ion beam in the accelerator is accelerated (step S3). The ion beam extracted from the linear accelerator 14 is injected into the circular beam duct 4 which is a circular orbit of the synchrotron accelerator 3 through the injector 5 and circulates in the annular beam duct 4. The accelerator-and-transport-system control apparatus 39 slowly increases each excitation current of each bending magnet 6 and each quadrupole magnet 7 of the synchrotron accelerator 3 to the respective excitation currents corresponding to the energy $E_1$ in order to increase the energy of the injected ion beam to the energy $E_1$ and slowly increases the radiofrequency voltage applied from the radiofrequency power supply to the acceleration apparatus 8. The ion beam is accelerated in correspondence with an increase in the radiofrequency voltage applied from the acceleration apparatus 8 during circulating in the beam duct 4 and the energy of the ion beam rises soon to the energy $E_1$ necessary for the ion beam to reach the layer $L_1$. When the energy of the ion beam becomes the energy $E_1$, the acceleration of the ion beam by the acceleration apparatus 8 is stopped. The ion beam holding the energy $E_1$ circulates in the annular beam duct 4.

In each of the processes of steps S4 and S6 to S18 (including steps S6A to S6E shown in FIG. 3) described below, each program (or one program) for executing these processes is stored in the memory 60 of the scanning control apparatus 40. These programs are executed by the scanning control apparatus 40, concretely, a concerned apparatus among above-mentioned the irradiation position control apparatus 52, the dose determination apparatus 53, the layer determination apparatus 54, and the beam position monitoring apparatus 55 (including the error operating apparatus 56 and the error determination apparatus 57) which are included in the scanning control apparatus 40.

The scanning magnet is controlled and the ion beam irradiation position is set to the target position P of the irradiation spot (step S4). The irradiation position control apparatus 52 controls the excitation currents supplied to the scanning magnets 28 and 29 based on the information of the target position (center position) $P_{i,j}$ of the respective irradiation spots $A_{i,j}$ of each layer $L_i$, the information of the target position being stored in the memory 60 of the scanning control apparatus 40 and permits the scanning magnets 28 and 29 to generate bending electromagnetic force so that the target position $P_{i,j}$ is irradiated with the ion beam. The scanning magnet 28, concretely, the bending electromagnetic force generated by the scanning magnet 28 controls the position of the ion beam extracted from the synchrotron accelerator 3 at step S5 which will be described later in the y direction. The scanning magnet 29, concretely, the bending electromagnetic force generated by the scanning magnet 29 controls the position of the ion beam extracted from the synchrotron accelerator 3 in the x direction orthogonal to the y direction. Firstly, the irradiation position control apparatus 52 controls the excitation current supplied to the scanning magnets 28 and 29 so as to permit the ion beam to reach the target position (central position) $P_{1,1}$ ($x_{1,1}$, $y_{1,1}$) of the first irradiation spot $A_{1,1}$ in the layer $L_1$ and adjusts the bending electromagnetic force generated in the scanning magnets 28 and 29.

The irradiation position control apparatus 52 outputs a beam irradiation start signal when it determines that the excitation current supplied to the scanning magnets 28 and 29 has been controlled so that the ion beam reaches the target position $P_{i,j}$ of the irradiation spot $A_{i,j}$.

The ion beam is extracted from the accelerator (step S5). The beam irradiation start signal output from the irradiation position control apparatus 52 is input to the accelerator-and-transport-system control apparatus 39. The accelerator-and-transport-system control apparatus 39 closes the open/close switch 12 based on the beam irradiation start signal. Thus, the radiofrequency from the radiofrequency power supply 11 is applied to the ion beam circulating in the annular beam duct 4 from the extraction radiofrequency electrode 10. The ion beam circulating moves outside stable limit by the application of the radiofrequency and is extracted from the synchrotron accelerator 3 through the extraction deflector 13. The excitation current supplied to the extraction deflector 13 is also adjusted to the excitation current corresponding to the energy $E_1$ by the accelerator-and-transport-system control apparatus 39.

Each of the bending magnets 17, 19, and 20 and the quadrupole magnets 21, 22, and 23 of the beam transport system 15 is excited by the excitation currents determined by the energy $E_1$, so that the ion beam extracted from the synchrotron accelerator 3 is injected to the irradiation nozzle 27 through the beam path 16. This ion beam is scanned by the aforementioned bending electromagnetic force generated in each of the scanning magnets 28 and 29 and thus, the target position $P_{1,1}$ ($x_{1,1}$, $y_{1,1}$) of the irradiation spot $A_{1,1}$ in the layer $L_1$ of the target volume is irradiated with the ion beam.

The determination of the systematic error and random error is executed (step S6). The determination process of step S6 includes each process of steps S6A to 6E shown in FIG. 3 and each process of steps S6A to 6E will be explained referring to FIG. 3.

The actual irradiation position $Pa_{i,j}$ of the irradiation spot is input (step S6A). The beam position monitor 30 installed in the irradiation nozzle 27 measures the actual irradiation position $Pa_{1,1}$ ($x_{1,1}'$, $y_{1,1}'$) of the ion beam which is scanned by the scanning magnets 28 and 29 and with which the target position $P_{1,1}$ ($x_{1,1}$, $y_{1,1}$) of the irradiation spot $A_{1,1}$ is irradiated. The actual irradiation position $Pa_{1,1}$ ($x_{1,1}'$, $y_{1,1}'$) of the measured ion beam is input to the error operating apparatus 56 included in the beam position monitoring apparatus 55 of the scanning control apparatus 40 and is stored in the memory 60 of the scanning control apparatus 40.

The deviation $D_j$ between the target position $P_{i,j}$ of the irradiation spot $A_{i,j}$ and the actual irradiation position $Pa_{i,j}$ of the irradiation spot $A_{i,j}$ is calculated (step S6B). The error operating apparatus 56 substitutes the target position $P_j$ and the actual irradiation position $Pa_j$ into formula (1) using a certain layer $L_i$ as a subject and obtains the deviation $D_j$. In the explanation at steps S6B to S6E, the additional letter portion of "i (i = 1, 2, ..., m)" showing the No. of the layer is omitted in the irradiation spot $A_{i,j}$, the target position $P_{i,j}$, the actual irradiation position $Pa_{i,j}$, the systematic error $Es_{i,j}$, and the random error $Er_{1,1}$.

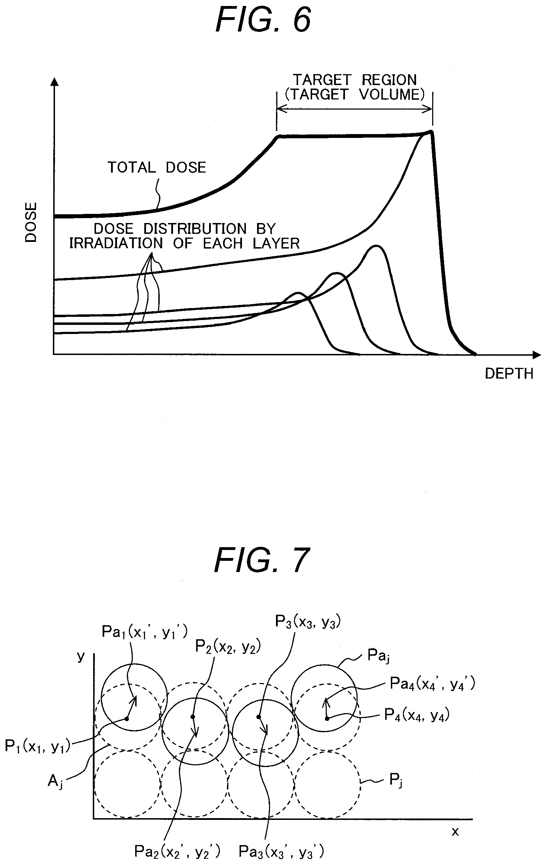

For example, in the layer $L_1$, as shown in FIG. 7, the actual irradiation position $Pa_1$ ($x_1'$, $y_1'$), the actual irradiation position $Pa_2$ ($x_2'$, $y_2'$), and the actual irradiation position $Pa_3$ ($x_3'$, $y_3'$) for the target position $P_1$ ($x_1$, $y_1$) of the irradiation spot $A_1$, the target position $P_2$ ($x_2$, $y_2$) of the irradiation spot $A_2$, and the target position $P_3$ ($x_3$, $y_3$) of the irradiation spot $A_3$ are assumed to have been measured by the beam position monitor 30. The deviation $D_1$ between the target position $P_1$ and the actual irradiation position $Pa_1$ becomes $Dx_1$ ($= x_1 - x_1'$) and $Dy_1$ ($= y_1 - y_1'$), and the deviation $D_2$ between the target position $P_2$ and the actual irradiation position $Pa_2$ becomes $Dx_2$ ($= x_2 - x_2'$) and $Dy_2$ ($= y_2 - y_2'$), and the deviation $D_3$ between the target position $P_3$ and the actual irradiation position $Pa_3$ becomes $Dx_3$ ($= x_3 - x_3'$) and $Dy_3$ ($= y_3 - y_3'$). Similarly, the $D_j$ (j = 4, 5, ..., n) from the deviation $D_4$ afterward is obtained. Further, $Dx_j$ is a deviation between the target position $P_j$ and the actual irradiation position $Pa_j$ in the x direction and $Dy_j$ is a deviation between the target position $P_j$ and the actual irradiation position $Pa_j$ in the y direction.

The actual irradiation position $Pa_2$ ($x_2'$, $y_2'$) and $Dx_2$ ($= x_2 - x_2'$) and $Dy_2$ ($= y_2 - y_2'$) which are the deviation $D_2$ are obtained for the irradiation spot $A_2$ which is irradiated next with the ion beam after it finishes that the irradiation spot $A_1$ is irradiated with the ion beam in the layer $L_1$. Further, the actual irradiation position $Pa_3$ ($x_3'$, $y_3'$) and $Dx_3$ ($= x_3 - x_3'$) and $Dy_3$ ($= y_3 - y_3'$) which are the deviation $D_3$ are obtained for the irradiation spot $A_3$ which is irradiated next with the ion beam after it finishes that the irradiation spot $A_2$ is irradiated with the ion beam in the layer $L_1$. However, to assist the understanding, here, they are explained together with the actual irradiation position $Pa_1$ of the irradiation spot $A_1$ and the deviation $D_1$. Also for the systematic error Es and the random error Er at step S6C which will be described later, the similar explanation is performed.

The systematic error Esi,j and the random error $Er_{i,j}$ are calculated (step S6C). The error operating apparatus 56 calculates the systematic error $Es_j$ and the random error $Er_j$. The systematic error $Es_j$ obtains the systematic error $Esx_j$ in the x direction and the systematic error $Esy_j$ in the y direction. The systematic errors $Esx_j$ and $Esy_j$ for the target positions $P_1$ ($x_1$, $y_1$), $P_2$ ($x_2$, $y_2$), and $P_3$ ($x_3$, $y_3$) of the irradiation spots $A_1$, $A_2$, and $A_3$ of the layer $L_1$ shown in FIG. 7 are as shown in Table 1.

Table 1

| Systematic error of irradiation spot | | |
|---|---|---|
| Irradiation spot $A_j$ | Systematic error $Esx_j$ in x direction | Systematic error $Esy_j$ in y direction |
| $A_1$ | $Esx_1 = Dx_1$ | $Esy_1 = Dy_1$ |
| $A_2$ | $Esx_2 = (Dx_1 + Dx_2) /2$ | $Esy_2 = (Dy_1 + Dy_2) /2$ |
| $A_3$ | $Esx_3 = (Dx_1 + Dx_2 + Dx_3) /3$ | $Esy_3 = (Dy_1 + Dy_2 + Dy_3) /3$ |

The random error $Er_j$ also obtains the random error $Erx_j$ in the x direction and the random error $Ery_j$ in the y direction. The random errors $Erx_j$ and $Ery_j$ for the target positions $P_1$ ($x_1$, $y_1$), $P_2$ ($x_2$, $y_2$), and $P_3$ ($x_3$, $y_3$) of the irradiation spots $A_1$, $A_2$, and $A_3$ of the layer $L_1$ shown in FIG. 7 are as shown in Table 2.

Table 2

| Random error of irradiation spot | | |
|---|---|---|
| Irradiation spot $A_j$ | Random error $Erx_j$ in x direction | Random error $Ery_j$ in y direction |
| $A_1$ | $Erx_1 = x_{1'} - Dx_1$ | $Ery_1 = y_{1'} - Dy_1$ |
| $A_2$ | $Erx_2 = x_{2'} - (Dx_1 + Dx_2)/2$ | $Ery_2 = y_{2'} - (Dy_1 + Dy_2)/2$ |
| $A_3$ | $Erx_3 = x_{3'} - (Dx_1 + Dx_2 + Dx_3)/3$ | $Ery_3 = y_{3'} - (Dy_1 + Dy_2 + Dy_3)/3$ |

Whether the systematic error Esi,j exists in the first permissible range is determined (Step S6D). The systematic error $Es_j$ and the random error $Er_j$ which are obtained by the error operating apparatus 56 are input to the error determination apparatus 57. The error determination apparatus 57 firstly determines whether the systematic error $Es_j$ exists in the first permissible range.

The first permissible range is the permissible range As of the systematic error Esi,j shown in FIG. 10. The permissible range As is demarcated by a lower limit value -As and an upper limit value +As based on the target position $P_j$ of the irradiation spot $A_j$. When the systematic error $Es_j$ is within the lower and the upper limit, it is determined that the systematic error Esj exists within the first permissible range. Namely, the determination at step S6D is "Yes". When the systematic error $Es_j$ is outside of the upper or the lower limit, it is determined that the systematic error $Es_j$ has deviated from the first permissible range, and the determination at step S6D is "No".

Concretely, the permissible range As includes a lower limit value -Asx and an upper limit value +Asx of the permissible range Asx in the x direction and a lower limit value -Asy and an upper limit value +Asy of the permissible range Asy in the y direction. Therefore, the determination of whether the systematic error $Es_j$ exists within the first permissible range is performed in the x and y direction.

The permissible range As (concretely, Asx and Asy) of the systematic error Es is stored in the memory 60 of the scanning control apparatus 40.

When the systematic error $Esx_j$ is within the upper and lower limit), it is determined that the systematic error $Es_j$ exists within the first permissible range. Namely, the determination at step S6D is "Yes". When the systematic error $Es_j$ deviates from the first permissible range, and the determination at step S6D is "No".

When the determination at step S6D is "No", a beam irradiation stop signal is output (step S18). When it is determined that the systematic error $Es_j$ has deviated from the first permissible range, the error determination apparatus 57 outputs the beam irradiation stop signal.

The ion beam irradiation is stopped (step S19). The beam irradiation stop signal output from the error determination apparatus 57 is input to the accelerator-and-transport-system control apparatus 39. The accelerator-and-transport-system control apparatus 39 inputting the beam irradiation stop signal outputs an opening signal to the open/close switch 12 and a closing signal to the shutter 24. An actuator of the open/close switch 12 inputting the opening signal opens the open/close switch 12, and an actuator of the shutter 24 inputting the closing signal closes the shutter 24. When the open/close switch 12 is opened, the application of the radiofrequency to the extraction radiofrequency electrode 10 is stopped and the extraction of the ion beam circulating in the beam duct 4 from the synchrotron accelerator 3 is stopped. When the shutter 24 is closed, even if the ion beam is extracted from the synchrotron accelerator 3, the ion beam is interrupted by the shutter 24 and does not reach the irradiation nozzle 27. The accelerator-and-transport-system control apparatus 39 furthermore stops the linear accelerator 14 (or the ion source).

As seen above, when the systematic error Esi,j deviates from the first permissible range, the irradiation of the ion beam to the target volume of the patient 34 is stopped. When the systematic error Esi,j deviates from the first permissible range, even if any one of the application stop of the radiofrequency to the extraction radiofrequency electrode 10, the interruption of the beam path 16 by the shutter 24, and the stop of the linear accelerator 14 (or the ion source) is executed, the irradiation of the ion beam to the target volume of the patient 34 is stopped.

When the determination at step S6D is "Yes", whether the random error Eri,j exists within the second permissible range is determined (step S6E). The error determination apparatus 57 determines whether the random error Eri,j input from the error operating apparatus 56 exists within the second permissible range.

The second permissible range is the permissible range Ar of the random error Eri,j shown in FIG. 10. The permissible range Ar is demarcated by a lower limit value -Ar and an upper limit value +Ar based on the mean position Pmj of the actual irradiation position $Pa_j$ of the irradiation spot $A_j$. When the random error $Er_j$ is within the lower and upper limit, it is determined that the random error $Er_j$ exists within the second permissible range. Namely, the determination at step S6E is "Yes". When it is determined that the random error $Er_j$ has deviated from the second permissible range and the determination at step S6E is "No".

Concretely, the permissible range Ar includes a lower limit value - Arx and an upper limit value +Arx of the permissible range Arx in the x direction and a lower limit value - Ary and an upper limit value +Ary of the permissible range Ary in the y direction. Therefore, the determination of whether the random error $Er_{i,j}$ exists within the second permissible range is performed for the x and y direction.

The permissible range Ar of the random error Er (concretely, Arx and Ary) is stored in the memory 60 of the scanning control apparatus 40.

When the random error $Erx_j$ in the x direction and the random error $Ery_j$ in the y direction are within the lower and upper limit, respectively, it is determined that the random error $Er_{i,j}$ exists within the second permissible range. Namely, the determination at step S6E is "Yes". When it is determined that the random error $Er_{i,j}$ does not exist within the second permissible range, that is, that random error $Er_{i,j}$ has deviated from the second permissible range, and the determination at step S6E is "No". When the determination at step S6E is "No", at the step S18 mentioned above, the error determination apparatus 57 outputs the beam irradiation stop signal. The beam irradiation stop signal is input to the accelerator-and-transport-system control apparatus 39.

When the random error $Er_{i,j}$ deviates from the second permissible range, similarly to the case when the systematic error Esi,j deviates from the first permissible range, the accelerator-and-transport-system control apparatus 39 inputting the beam irradiation stop signal executes the application stop of the radiofrequency to the extraction radiofrequency electrode 10, and the interruption of the beam path 16 by the shutter 24, and the irradiation of the ion beam to the target volume is stopped (step S19).

Whether the dose $R_{i,j}$ of the irradiation spot $A_{i,j}$ has become the target dose $R0_{i,j}$ is determined (step S7) (refer to FIG.2B). When the determination at step S6E is "Yes", that is, when the systematic error $Es_{i,j}$ exists within the first permissible range As and the random error $Er_{i,j}$ exists within the second permissible range Ar, the dose determination apparatus 53 determines the dose $R_{i,j}$. The dose monitor 31 measures the dose Ri,j of the actual irradiation position $Pa_{i,j}$ from the point of time when the ion beam irradiation to the actual irradiation position $Pa_{i,j}$ of the irradiation spot $A_{i,j}$ starts. The measured dose $R_{i,j}$ at the actual irradiation position $Pa_{i,j}$ is input to the dose determination apparatus 53 of the scanning control apparatus 40. The dose determination apparatus 53 determines whether the dose $R_{i,j}$ has become the target dose $R0_{i,j}$.

When the dose $R_{i,j}$ does not reach the target dose $R0_{i,j}$, that is, when the determination at step S7 is "No", the irradiation of the ion beam is continued (step S8). Concretely, the irradiation of the ion beam to the actual irradiation position $Pa_{i,j}$ is continued.

Thereafter, whether the dose $R_{i,j}$ of the irradiation spot $A_{i,j}$ has become the target dose $R0_{i,j}$ is determined (step S9). The determination at step S9 is similar to the determination at step S7 and is executed by the dose determination apparatus 53. When the determination at step S9 is "No", each process of steps S8 and S9 is repeated until the determination at step S9 becomes "Yes", that is, until the dose $R_{i,j}$ of the irradiation spot $A_{i,j}$ becomes the target dose $R0_{i,j}$.

When the determination at step S9 becomes "Yes", the beam irradiation stop signal is output (step S9A). When the dose $R_{i,j}$ of the irradiation spot $A_{i,j}$ becomes the target dose $R0_{i,j}$, the dose determination apparatus 53 outputs the beam irradiation stop signal. This beam irradiation stop signal is input to the accelerator-and-transport-system control apparatus 39.

The ion beam irradiation to the irradiation spot $A_{i,j}$ is stopped (step S10). The accelerator-and-transport-system control apparatus 39 inputting the beam irradiation stop signal outputs an opening signal to the open/close switch 12. By the opening signal, the open/close switch 12 opens and the application of the radiofrequency to the extraction radiofrequency electrode 10 is stopped. Therefore, the extraction of the ion beam from the synchrotron accelerator 3 is stopped and the ion beam irradiation to the irradiation position $Pa_{i,j}$ of the target volume is stopped. Even when the determination at step S7 becomes "Yes", at step S9A, the beam irradiation stop signal is output from the dose determination apparatus 53 to the accelerator-and-transport-system control apparatus 39.

Whether the irradiation to the layer $L_i$ has finished is determined (step S11). The layer determination apparatus 54 determines whether the irradiation spot $A_{i,j}$ which is not irradiated with ion beam does not exist on the layer $L_i$. Although the ion beam irradiation to the irradiation spot $A_{1,1}$ finished, the irradiation of the ion beam to the irradiation spot $A_{1,2}$, $A_{1,3}$, ..., $A_{1,j}$ in the layer $L_1$ still remains, so that the determination at step S11 becomes "No".

Accordingly, the information of the deviation $D_j$ in the irradiation position is stored (step S13). The deviation $D_1$ of the irradiation position of the irradiation spot $A_{1,1}$ is stored in the memory 60 of the scanning control apparatus 40 by the layer determination apparatus 54 because of necessity for each calculation of the systematic error Es and the random error Er in the next irradiation spot $A_{1,2}$ in the layer $L_1$.

j = j + 1 is executed (step S14). The irradiation position control apparatus 52 replaces "j" with "j+1". By doing this, the next irradiation spot $A_{i,j+1}$, for example, the ion beam irradiation to the target position $P_{1,2}$ of the irradiation spot $A_{1,2}$ is enabled.

Whether the circulating ion beam is available is determined (step S15). The accelerator-and-transport-system control apparatus 39 determines whether the irradiation to the target position $P_{1,2}$ of the next irradiation spot $A_{1,2}$ is enabled by the ion beam circulating in the circular beam duct 4 which is a circular orbit when the irradiation to the target position $P_{1,2}$ of the irradiation spot $A_{1,2}$ finishes, that is, when the closing signal is output to the open/close switch 12. When the ion beam in an amount capable of completing the irradiation to the target position $P_{1,2}$ of the irradiation spot

$A_{1,2}$ is circulating in the beam duct 4, the determination at step S15 becomes "Yes". The target position $P_{1,2}$ of the next irradiation spot $A_{1,2}$ also exists in the same layer $L_1$ as that of the target position $P_{1,1}$ of the preceding irradiation spot $A_{1,1}$, so that the process of step S4 is executed by the irradiation position control apparatus 52. After the respective bending electromagnetic forces of the scanning magnets 28 and 29 are adjusted by the process of step S4, the irradiation position control apparatus 52 outputs the beam irradiation start signal to the accelerator-and-transport-system control apparatus 39. The accelerator-and-transport-system control apparatus 39 inputting the beam irradiation start signal outputs the closing signal for closing the open/close switch 12. As a result, the radiofrequency is applied to the extraction radiofrequency electrode 10 and the extraction of the ion beam from the synchrotron accelerator 3 is started.

When the amount of the ion beam circulating is small and the amount of the ion beam is insufficient in the completion of irradiation to the target position $P_{1,2}$ of the irradiation spot $A_{1,2}$, the linear accelerator 14 is started at step S2 and the ion beam is supplied from the linear accelerator 14 to the synchrotron accelerator 3. Furthermore, the acceleration of the ion beam at step S3 is performed. Thereafter, the process at each step to be executed when the determination at step S15 becomes "Yes" which will be described below is executed.

Assume that the determination at Step S15 has become "Yes". Each of the processes step S4 (adjustment of the bending electromagnetic force generated in each of the scanning magnets 28 and 29 to irradiate the ion beam to the target position $P_{1,2}$) and step S5 (extraction of the ion beam from the synchrotron accelerator 3) is executed and the target position $P_{1,2}$ of the irradiation spot $A_{1,2}$ of the layer $L_1$ is irradiated with the ion beam. Thereafter, as mentioned above, each of the processes steps S6 to S9, S9A, S10, and S11 is executed (FIGs. 2A and 2B). At step S6, as mentioned above, each of the processes steps S6A to S6E (if necessary, steps S18 and S19) is executed. At this time, at step S6B, $Dx_2$ (= $x_2$ - $x_2$') and $Dy_2$ (= $y_2$ - $y_2$') which are the aforementioned deviation $D_2$ are obtained and at step 6C, the systematic error $Es_2$ (the systematic errors $Esx_2$ and $Esy_2$) and the random error $Er_2$ (the random errors $Erx_2$ and $Ery_2$) for the irradiation spot $A_2$ in the layer $L_1$ are obtained. However, when the determination at step S6D or step S6E is "No", the output of the beam irradiation stop signal at step S18 and the irradiation stop of the ion beam at step S19 are performed.

Each of the processes steps S13 to S15, S2 to S9, S9A, S10, and S11 (or steps S13 to S15, S4 to S9, S9A, S10, and S11) which are described above is repeated until the ion beam irradiation to all the irradiation spots $A_{i,j}$ in the layer $L_1$ is performed and the determination at step S11 becomes "Yes". When the determination at step S11 becomes "Yes", the process step S12 is executed.

The information of the deviation $D_j$ in the irradiation position is deleted (step S12). The layer determination apparatus 54 deletes the information of all the deviations $D_j$ in the layer $L_1$ stored in the memory 60 of the scanning control apparatus 40 at step S13. The layer determination apparatus 54 determines whether the ion beam irradiation to the target positions $P_{i,j}$ of all the irradiation spots $A_{i,j}$ in all the layers of the target volume has finished (step S16). The ion beam irradiation to all the irradiation spots $A_{i,j}$ in the layer $L_1$ just finished, so that the determination at step S16 performed by the layer determination apparatus 54 becomes "No".

At this time, the accelerator-and-transport-system control apparatus 39 slowly reduces each excitation current of each bending magnet 6 and each quadrupole magnet 7 of the synchrotron accelerator 3 and also slowly reduces the radiofrequency voltage applied to the acceleration apparatus 8. The ion beam circulating in the beam duct 4 reduces the speed. Furthermore, i = i + 1 is executed (step S17). The irradiation position control apparatus 52 replaces "i" with "i+1". By doing this, the ion beam irradiation to the target position $P_{i,j}$ of the irradiation spot $A_{i,j}$ in the next layer $L_{i+1}$, for example, the layer $L_2$ is enabled. As mentioned above, the accelerator-and-transport-system control apparatus 39 executes each of the processes steps S2 and S3. In the process of step S3, the accelerator-and-transport-system control apparatus 39 slowly increases each excitation current of each bending magnet 6 and each quadrupole magnet 7 of the synchrotron accelerator 3 and also slowly increases the radiofrequency voltage applied to the acceleration apparatus 8. By doing this, the energy of the ion beam circulating in the beam duct 4 is accelerated up to the energy $E_2$ necessary for the ion beam to reach the layer $L_2$. Thereafter, each step from step S4 afterward is executed successively and as mentioned above, each target position Pi,j in the layer $L_2$ is irradiated successively with the ion beam. When the determination at step S16 becomes "Yes", it finishes that the target volume of the patient 34 lying on the treatment bed 33 is irradiated with the ion beam. Namely, the treatment of the patient 34 finishes.

When the treatment of the patient 34 finishes, the dose distribution in the depth direction of the target volume (target region) becomes the distribution of the total dose shown in FIG. 6 and in the target volume, a uniform dose is irradiated in the depth direction. The total dose distribution is the total of the dose distribution by the irradiation to each layer $L_i$. Further, the dose distribution in the section of the target volume in the direction perpendicular to the ion beam irradiation direction becomes more uniform.

Here, the permissible range As of the systematic error Es and the permissible range Ar of the random error Er which are stored in the memory 60 will be explained. As mentioned above, the permissible range As is set so as to include the upper limit value (+Asx, +Asy) and the lower limit value (-Asx, -Asy) in the x direction and y direction based on the target position Pi,j of the irradiation spot $A_{i,j}$ (refer to FIG. 10). During the period from the first determination at step S6D which is performed from the irradiation spot $A_{i,j}$ (for example, the irradiation spot $A_{1,1}$ of the layer $L_1$) which

was first irradiated with the ion beam till the determination count at step S6D reaches h times (h < j), as shown in FIG. 4, the permissible range As of the systematic error Es used for the determination at step S6D is set more widely than the permissible range As of the systematic error Es used for the determination at step S6D when the determination count is h+1 times or more at step S6D .

As seen above, the reason that the permissible range As used for the determination at step S6D when determination count is 1 to h times is set widely is as described below. For example, after switching of the irradiation spot which is irradiated with the ion beam from the irradiation spot $A_{1,1}$ of the layer $L_1$ which was first irradiated with the ion beam to the irradiation spot $A_{1,2}$ of the layer $L_1$, in the determination results of several times at step S6D, the accuracy of the mean position Pmi,j of the actual irradiation position Pai,j obtained gets worse due to the scattering of the random error Er. For this reason, the permissible range As used for the determination at step S6D when determination count is h times or lower is set widely as mentioned above. Further, the permissible range As used for the determination when the determination count is h times or lower is set, for example, to 150% of the permissible range As used for the determination when the determination count is h+1 times or more.

As mentioned above, the permissible range Ar of the random error Er is set so as to include the upper limit values (+Arx, +Ary) and the lower limit values (-Arx, - Ary) in the x direction and y direction based on the mean position Pmi,j of the actual irradiation position Pai,j (refer to FIG. 10). The permissible range Ar of the random error used in the determination at sep S6E when the determination count is h times or lower, as shown in FIG. 4, is also spread than the permissible range Ar used for the determination at step S6E when the determination count is h+1 times or more due to the same reason as that when the permissible range As of the systematic error Es is spread. The permissible range Ar used in the determination when the determination count is h times or lower is set to, for example, 150% of the permissible range Ar used for the determination when the determination count is h+1 times or more.

As mentioned above, the permissible range As, in the x direction and y direction, includes the upper limit values (+Asx and +Asy) and the lower limit values (-Asx and -Asy), so that in the permissible range As used in the determination when the determination count is h times or lower, the respective absolute values of the first upper values (the first "+Asx" and the first "+Asy") and the first lower values (the first "-Asx", the first "-Asy") are larger than the respective absolute values of the second upper values (the second "+Asx" and the second "+Asy") and the second lower values (the second "-Asx", the second "-Asy") in the permissible range As used for the determination when the determination count is h+1 times or more. The same may be said with the permissible range Ar.

In the determination of the systematic error Es at step S6D when the determination count is h times or lower, the first upper limit value and the first lower limit value are used in both the x direction and y direction. In the determination of the systematic error Es at step S6D when the determination count is h+1 times or more, the second upper limit value and the second lower limit value are used in both the x direction and y direction.

In the determination of the random error Er at step S6E when the determination count is h times or lower, the first upper limit values (the first "+Arx" and the first "+Ary") of the permissible range Ar and the first lower limit values (the first "-Asx", the first "-Asy") of the permissible range Ar are used in both the x direction and y direction. In the determination of the random error Er at step S6E when the determination count is h+1 times or more, the second upper limit values (the second "+Arx" and the second "+Ary") of the permissible range Ar and the second lower limit values (the second "-Asx", the second "-Asy") are used in both the x direction and y direction.

In the present embodiment, the systematic error Es and the random error Er are obtained as an error of the actual irradiation position Pai,j of the irradiation sport Ai,j to the target position $P_{i,j}$ of the irradiation spot $A_{i,j}$ and the permissible range As of the systematic error Es and the permissible range Ar of the random error Er are set separately to determine the existence or no existence of deviation of the systematic error Es and the random error Er from the respective permissible errors, and these permissible ranges are used for those determinations. As a result, the permissible range As and the permissible range Ar can be set severely (narrowly), and the damage given to a healthy cell of the patient 34 can be reduced at the time of irradiation of the ion beam to the target volume, thus the safety improves.

Furthermore, the permissible range As and the permissible range Ar are set separately, so that even when the ion beam is thinned more in diameter, the probability that the systematic error deviates from the permissible range of the systematic error and furthermore the random error deviates from the permissible range of the random error is reduced. Therefore, the probability of irradiation stop of the ion beam to a patient is reduced extremely, and a stabler operation of the charged particle beam irradiation system is enabled, and unscheduled stop of the ion beam irradiation to the patient is reduced extremely. As a result, the number of persons capable of being treated per day can be increased.

In the present embodiment, whether the random error Er does not deviate from the permissible range Ar thereof is monitored, so that the dose distribution on the section in the direction perpendicular to the travelling direction of the ion beam can be made more uniform. Further, whether the systematic error Es does not deviate from the permissible range As thereof is monitored, so that whether the entire dose distribution is not deviated from the target volume outside the permissible range can be monitored.

When the systematic error Es deviates from the permissible range As or the random error Er deviates from the permissible range Ar, the irradiation of the ion beam to the patient 34 is stopped. Therefore, the safety to the patient 34 is improved.

Further, in the present embodiment, within the range in which the determination count of whether the systematic error Es deviates from the permissible range As is h times or lower, the permissible range As used for the determination performed within the range is made wider than the range of the permissible range As used for the determination when the determination count is h+1 times or more. Therefore, even when the accuracy of the mean position Pmi,j of the actual irradiation position Pai,j obtained gets worse due to the scattering of the random error Er, the probability of deviation of the systematic error Es from the permissible range As is reduced.

Further, in the present embodiment, within the range in which the determination count of whether the random error Er deviates from the permissible range Ar is h times or lower, the permissible range Ar used for the determination performed within the range is made wider than the range of the permissible range Ar used for the determination when the determination count is h+1 times or more. Therefore, similarly to the case of the systematic error Es, even when the accuracy of the mean position $Pm_{i,j}$ of the actual irradiation position Pai,j obtained gets worse, the probability of deviation of the random error Er from the permissible range Ar is reduced.

[Example being useful for the understanding of the invention]

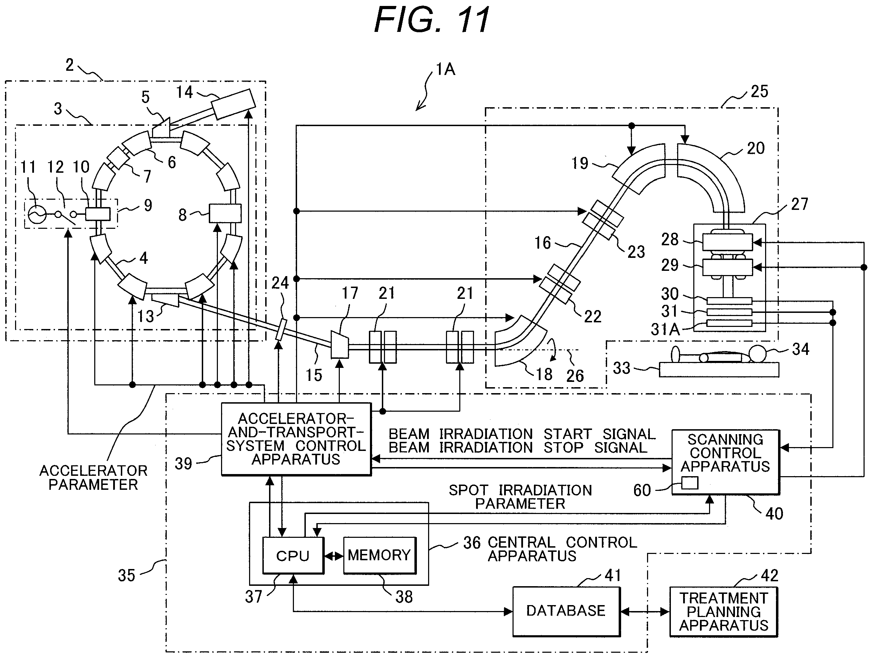

A charged particle beam irradiation system according to the example will be explained by referring to FIG. 11. A charged particle beam irradiation system 1A of the present example has a structure that in the charged particle beam irradiation system 1 of embodiment 1 shown in FIG. 1, the scanning control apparatus 40 is replaced with a scanning control apparatus 40A and a dose monitor 31A is added. The other structure of the charged particle beam irradiation system 1A is the same as that of the charged particle beam irradiation system 1. Among the two dose monitors, the dose monitor 31 measures the dose of each irradiation spot Ai,j which was irradiated with the ion beam, similarly to embodiment 1 and another dose monitor 31A measures the dose of the beam irradiation section $S_k$ described later.

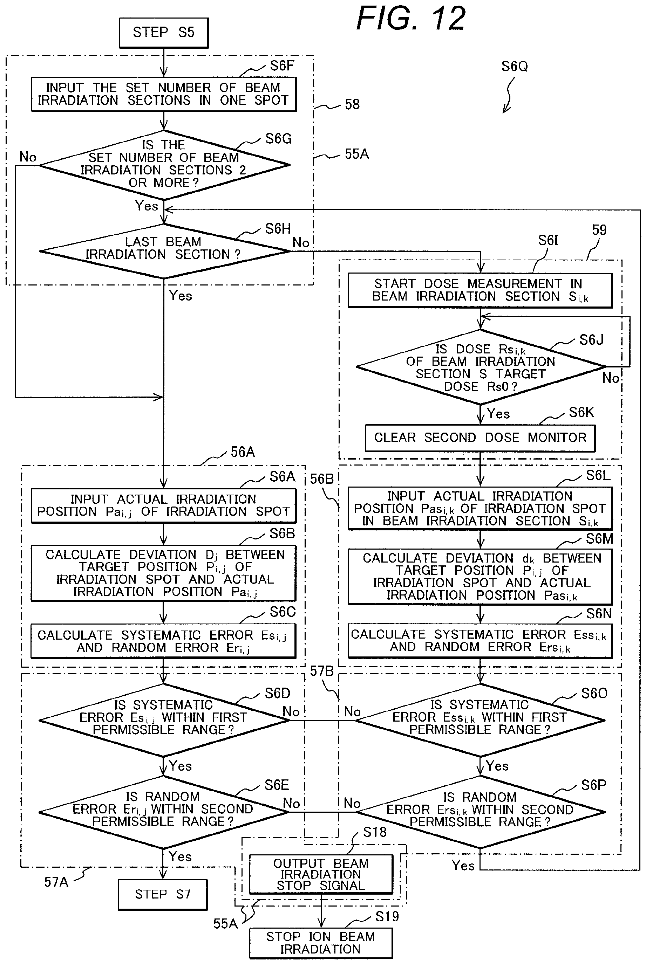

As shown in FIGs. 2A, 2B, and 12, the scanning control apparatus 40A includes the irradiation position control apparatus 52, the dose determination apparatus (the first dose determination apparatus) 53, the layer determination apparatus 54, a beam position monitoring apparatus 55A, and a dose determination apparatus (a second dose determination apparatus) 59. The beam position monitoring apparatus 55A includes an error operating apparatus (a first error operating apparatus) 56A, an error determination apparatus (a first error determination apparatus) 57A, an error operating apparatus (a second error operating apparatus) 56B, an error determination apparatus (a second error determination apparatus) 57B, and a beam irradiation section determination apparatus 58.

Further, in the charged particle beam irradiation system 1A, unlike the scanning control apparatus 40 of the charged particle beam system 1 of embodiment 1, the scanning control apparatus 40A executes the processes steps S4, S7 to S9, S9A, and S10 to S18 shown in FIGs. 2A and 2B and steps S6A to S6P included in step S6Q shown in FIG. 12. In the scanning control apparatus 40A of the charged particle beam irradiation system 1A, each process in Step S6Q shown in FIG. 12 is executed in place of each process in step S6 shown in FIG. 3. In the charged particle beam irradiation system 1A of the present example, a program of executing the processes steps S4 and S7 to S18 shown in FIGs. 2A and 2B and steps S6A to S6P shown in FIG. 12 is stored in the memory 60 of the scanning control apparatus 40A.

Among the processes steps S4, S6Q, S7 to S9, S9A, and S10 to S18 executed by the scanning control apparatus 40A, the processes steps S4, S14, and S17 are executed by the irradiation position control apparatus 52 (refer to FIG. 2A), and the processes steps S7 to S9 and S9A are executed by the dose determination apparatus 53 (refer to FIG. 2B), and the processes steps S11 to S13 and S16 are executed by the layer determination apparatus 54 (refer to FIG. 2B), and the processes steps S6A to S6C are executed by the error operating apparatus 56A (refer to FIG. 12), and the processes steps S6D, S6E, and S18 are executed by the error determination apparatus 57A (refer to FIG. 12), and the processes steps S6L to S6N are executed by the error determination apparatus 56B (refer to FIG. 12), and the processes steps S6O, S6P, and S18 are executed by the error determination apparatus 57B (refer to FIG. 12), and the processes steps S6F to S6H are executed by the beam irradiation section determination apparatus 58 (refer to FIG. 12), and the processes steps S6I, S6J, and S6K are executed by the dose determination apparatus 59 (refer to FIG. 12).

The processes steps S1 to S5, S7 to S9, S9A, S10 to S19, and S6A to S6E (some processes at Step S6Q) which are shown in FIGs. 2A, 2B, and 3 are executed even in the present example, similarly to embodiment 1. Among the processes at step S6Q shown in FIG. 12 which are not performed in embodiment 1, the processes steps S6F to S6P will be explained mostly. In the present example, the process step S6 executed in embodiment 1 is replaced with the procedure at step S6Q shown in FIG. 12.

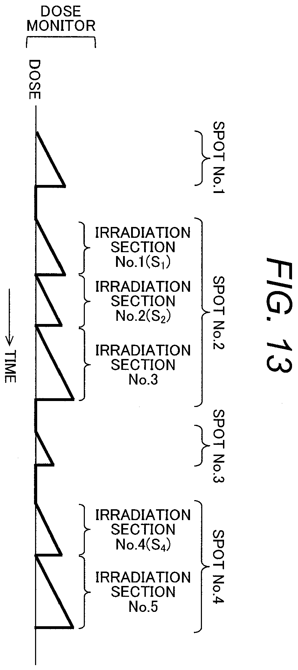

In a method of irradiating charged particle beam using the charged particle beam irradiation system 1A, a plurality of beam irradiation sections S (for example, the beam irradiation sections No. 1 to No. 5) are set for a plurality of irradiation spots (for example, the irradiation spots No. 2 and No. 4 shown in FIG. 13) of a part of a plurality of irradiation spots Ai,j set in the method of irradiating charged particle beam using the charged particle beam irradiation system 1 of embodiment 1. Concretely, the irradiation spot No. 2 includes three beam irradiation sections S (the beam irradiation sections No. 1 to No. 3) and the irradiation spot No. 4 includes two beam irradiation sections S (the beam irradiation sections No. 4 and No. 5) .

Each target dose R0 of the beam irradiation sections No. 1 ($S_1$), No. 2 ($S_2$), and No. 4 ($S_3$) and the irradiation spots No. 1 to No. 4 are preset using the treatment planning apparatus 42 before the ion beam irradiation. To make the irradiation dose distribution more uniform, in the irradiation spot with a large target dose R0, concretely, the irradiation spots No. 2 and No. 4, one beam irradiation section S is set so that the target dose RO becomes, for example, 0.033 MU. Here, an example is given where the target dose R0 is set to 0.033 MU. The respective target doses Rs0 in the beam irradiation sections No. 1, No. 2, and No. 4, that is, the beam irradiation sections $S_1$, $S_2$, and $S_4$, are 0.033 MU. In other words, the beam irradiation sections $S_1$, $S_2$, $S_3$, $S_4$,..., in the layer $L_i$ are the beam irradiation section $S_k$ (k = 1, 2, ..., p) with the target dose Rs0 set essentially to 0.033 MU. However, in the irradiation spot to which one beam irradiation section $S_k$ or a plurality of beam irradiation sections $S_k$ are set, when the beam irradiation section (for example, the beam irradiation sections No. 3 and No. 5) with the dose less than two times of 0.033 MU irradiated by the ion beam remains other than the beam irradiation sections $S_k$ set to 0.033 MU, the remained beam irradiation section is not divided into a plurality of beam irradiation sections $S_k$ and is kept as one beam irradiation section S. Further, for example, the irradiation spot with the target dose RO less than 0.033 MU are not divided into a plurality of beam irradiation sections S, too.

The target position $P_{i,j}$ which is irradiated with the ion beam in a plurality of beam irradiation sections S set in one irradiation spot Ai,j is the target position $P_{i,j}$ of the irradiation spot Ai,j. Even when the beam irradiation section which is irradiated with the ion beam changes from one beam irradiation section S (for example, the beam irradiation section $S_1$) to other beam irradiation section S (for example, the beam irradiation section $S_2$) in one irradiation spot $A_{i,j}$, the target position $P_{i,j}$ which is irradiated with the ion beam by the scanning magnets 28 and 29 is not changed and is kept in the target position $P_{i,j}$ of the irradiation spot Ai,j. For example, when the ion beam irradiation is changed from the beam irradiation section $S_1$ to the beam irradiation section $S_2$, the dose monitor 31A that has measured the dose of the beam irradiation section $S_1$ is reset and the dose monitor 31A measures the dose of the beam irradiation section $S_2$ from zero. In this way, the dose monitor 31A measures the dose for each beam irradiation section S in the irradiation spot Ai,j.

For example, the irradiation spots No. 1, No. 2, No. 3, and No. 4 shown in FIG. 13 are assumed as the irradiation spots $A_{1,11}$, $A_{1,12}$, $A_{1,13}$, and $A_{1,14}$ of the layer $L_1$. And, for simplicity of explanation, it is assumed that the irradiation spots $A_{1,1}$ to $A_{1,11}$ do not have a plurality of beam irradiation sections S set.

The method of irradiating charged particle beam using the charged particle beam irradiation system 1A of the present example will be explained below. In the method of irradiating charged particle beam of the present example, each of the processes steps S1 to S3 and S5 (refer to FIG. 2A) is executed by the accelerator-and-transport-system control apparatus 39. Furthermore, in the target positions from the target position $P_{1,1}$ of the irradiation spot $A_{1,1}$ to the target position $P_{1,11}$ of the irradiation spot $A_{1,11}$ (each target position from the irradiation spot $A_{1,1}$ to the irradiation spot $A_{1,11}$ do not have a plurality of beam irradiation sections S set), step S4 shown in FIGs. 2A and 2B, steps S6A to S6E included in step S6Q shown in FIG. 12, and steps S7 to S9, S9A, and S10 to S17 are executed repeatedly, similarly to embodiment 1 because the determination at step S6G (refer to FIG. 12) becomes "No". However, when the determination at step S6D or Step S6E is "No", the output of the beam irradiation stop signal at step S18 and the irradiation stop of the ion beam at step S19 are performed. In the present example, the procedure at step S6Q shown in FIG. 12 is executed between step S5 and step S7 in place of the aforementioned procedure shown in FIG. 3 at step S6.

In the ion beam irradiation for each of the target positions from the target position $P_{1,1}$ of the irradiation spot $A_{1,1}$ to the target position $P_{1,11}$ of the irradiation spot $A_{1,11}$, each process of steps S4, S6F, S6G, and S6A to S6E, S7 to S9, S9A, S10, and S11 are executed by the irradiation position control apparatus 52, the beam irradiation section determination apparatus 58, the error operating apparatus 56A, the error determination apparatus 57A, the dose determination apparatus 53, and the layer determination apparatus 54 including in the scanning control apparatus 40A. Step S4 is executed by the irradiation position control apparatus 52, and step S5 is executed by the accelerator-and-transport-system control apparatus 39, and then the number of set beam irradiation sections S in one irradiation spot is input (step S6F). The beam irradiation section determination apparatus 58 reads that set number of the beam irradiation sections S from the memory 60 of the scanning control apparatus 40. Next, whether the number of set beam irradiation sections S is 2 or larger is determined (step S6G). Since the beam irradiation sections S are not set in any of the irradiation spots $A_{1,1}$ to $A_{1,11}$, the determination at step S6G performed by the beam irradiation section determination apparatus 58 becomes "No". Thus, for each of the irradiation spots $A_{1,1}$ to $A_{1,11}$, each process of steps S6A to S6C described in embodiment 1 is executed by the error operating apparatus 56A and furthermore, each process of steps S6D and S6E described in embodiment 1 is executed by the error determination apparatus 57A. At this time, when the determination at step S6D or S6E becomes "No", the error determination apparatus 57A outputs the beam irradiation stop signal at step S18. The accelerator-and-transport-system control apparatus 39 inputting the beam irradiation stop signal executes the aforementioned control at step S19, so that the ion beam irradiation to the target volume of the patient 34 is stopped. When the determination at step S6D and S6E is "Yes", the dose determination apparatus 53 determines whether the dose $R_{i,j}$ of the actual irradiation position $Pa_{i,j}$ of the irradiation spot $A_{i,j}$ measured by the dose monitor 31 and the target dose $R0_{i,j}$ of the irradiation spot $A_{i,j}$ coincide with each other at steps S7 and S9.

When the ion beam irradiation to the target position $P_{1,11}$ of the irradiation spot $A_{1,11}$ finishes, the determination at step S11 by the layer determination apparatus 54 becomes "No" and each process of steps S13 to S15 is executed. According to the determination results at step S15, each process from step S2 or step S4 is executed similarly to embodiment 1. At step S4, the bending electromagnetic force generated in each of the scanning magnets 28 and 29 is adjusted by the irradiation position control apparatus 52 so that the irradiation position of the ion beam becomes the target position $P_{1,12}$ of the irradiation spot $A_{1,12}$.

The irradiation spot $A_{1,12}$ (the irradiation spot No. 2) includes three beam irradiation sections of No. 1 to No. 3. At step S6F, "3" is input as the number of set beam irradiation sections and the determination at step S6G becomes "Yes".

Whether the beam irradiation section is a last one is determined (step S6H). The beam irradiation section determination apparatus 58 determines whether the beam irradiation section $S_1$ which is a subject in the irradiation spot $A_{1,12}$ is the final beam irradiation section in the irradiation spot $A_{1,12}$. The beam irradiation section $S_1$ is the first beam irradiation section in the irradiation spot $A_{1,12}$, so that the determination at step S6H becomes "No". When the determination at step S6H becomes "No", each process of steps S6I to S6P is executed.

The measurement of the dose in the beam irradiation section $S_{i,k}$ is started (step S6I). The dose determination apparatus 59 permits the measurement of the dose in the beam irradiation section $S_{i,k}$ by the dose monitor 31A, concretely, in the first beam irradiation section $S_1$ in the irradiation spot $A_{1,12}$ which is irradiated with the ion beam start. The dose determination apparatus 59 determines whether the dose $Rs_1$ has become the target dose $Rs_0$ (for example, 0.033 MU) (step S6J). When the dose $Rs_1$ has not reached the target dose $Rs_0$, that is, when the determination at step S6J is "No", the ion beam irradiation is continued until the determination at step S6J becomes "Yes".

The second dose monitor is cleared (step S6K). When the dose $Rs_1$ reaches the target dose $Rs_0$ and the determination at step S6J becomes "Yes", the dose monitor 31A that has measured the dose $Rs_1$ in the beam irradiation section $S_1$ is cleared to zero.

The actual irradiation position $Pas_{i,k}$ of the irradiation spot in the beam irradiation section $S_{i,k}$ of the irradiation spot is input (step S6L). The beam position monitor 30 installed on the irradiation nozzle 27 measures the actual irradiation position $Pas_{i,k}$ ($xs_{1,1}'$, $ys_{1,1}'$) which is irradiated with the ion beam to the target position $P_{1,12}$ ($x_{1,12}$, $y_{1,12}$) of the irradiation spot $A_{1,12}$ in the beam irradiation section $S_1$ in the irradiation spot $A_{1,12}$. The error operating apparatus 56B inputs the information of the actual irradiation position and stores it in the memory 60 of the scanning control apparatus 40.

The deviation $d_k$ between the target position $P_{i,j}$ of the irradiation spot $A_{i,j}$ and the actual irradiation position $Pas_{i,k}$ in the beam irradiation section $S_{i,k}$ in the irradiation spot $A_{i,j}$ is calculated (step S6M). Using a certain layer $L_i$ as a subject, the error operating apparatus 56B substitutes the target position $P_j$ and the actual irradiation position $Pas_k$ in the beam irradiation section $S_k$ into formula (11) and obtains the deviation $d_k$ ($k = 1, 2, ..., p$). In the explanation at steps S6K to S6P and formulas (11) to (20), the additional letter portion of "i ($i = 1, 2, ..., m$)" indicating the layer No. is omitted in the irradiation spot $A_{i,j}$, the target position $P_{i,j}$, the actual irradiation position $Pas_{i,j}$, the systematic error $Ess_{i,j}$, and the random error $Ers_{i,j}$.

$$d_k = P_j - Pas_k \qquad \cdots (11)$$

For example, in the layer $L_i$, in the irradiation spot $A_{12}$ of the target position $P_{12}$ ($x_{12}$, $y_{12}$), the actual irradiation position $Pas_1$ ($xs_1'$, $ys_1'$) in the beam irradiation section $S_1$ is assumed to have been measured by the beam position monitor 30. The deviation $d_1$ between the target position $P_1$ and the actual irradiation position $Pas_1$. becomes $dx_1$ (= $xs_1$ - $xs_1'$) and $dy_1$ (= $ys_1$ - $ys_1'$). The $dx_k$ is the deviation between the target position $P_j$ and the actual irradiation position $Pas_k$ in the x direction and the $dy_k$ is the deviation between the target position $P_j$ and the actual irradiation position $Pas_k$ in the y direction. The calculated deviation $d_k$ (concretely, the deviation $d_1$) is stored in the memory 60 of the scanning control apparatus 40.

The systematic error $Ess_{i,k}$, and the random error $Ers_{i,k}$ are calculated (step S6N). The error operating apparatus 56B calculates the systematic error $Ess_k$ and the random error $Ers_k$.

As a systematic error $Ess_k$, the systematic error $Essx_k$ in the x direction and the systematic error $Essy_k$ in the y direction are obtained. The systematic errors $Essx_1$ and $Essy_1$ in the beam irradiation section $S_1$ are as shown in Table 3.

Table 3

| Systematic error in beam irradiation section | | |
|---|---|---|
| Beam irradiation section $S_k$ | Systematic error $Essx_j$ in x direction | Systematic error $Essy_j$ in y direction |
| $S_1$ | $Essx_1 = dx_1$ | $Essy_1 = dy_1$ |
| $S_2$ | $Essx_2 = (dx_1 + dx_2)/2$ | $Essy_2 = (dy_1 + dy_2)/2$ |

(continued)

| Systematic error in beam irradiation section | | |
|---|---|---|
| Beam irradiation section $S_k$ | Systematic error $Essx_j$ in x direction | Systematic error $Essy_j$ in y direction |
| $S_3$ | $Essx_3 = (dx_1 + dx_2 + dx_3)/3$ | $Essy_3 = (dy_1 + dy_2 + dy_3)/3$ |

The random error $Ers_k$ is obtained. As for also the random error $Ers_k$, the random error $Ersx_k$ in the x direction and the random error $Ersy_k$ in the y direction are obtained respectively. The random errors $Ersx_1$ and $Ersy_1$ in the beam irradiation section $S_1$ are respectively as shown in Table 4.

Table 4

| Random error in beam irradiation section | | |
|---|---|---|
| Beam irradiation section $S_k$ | Random error $Ersx_j$ in x direction | Random error $Ersy_j$ in y direction |
| $S_1$ | $Ersx_1 = xs_1' - dx_1$ | $Ersy_1 = ys_1' - dy_1$ |
| $S_2$ | $Ersx_2 = xs_2' - (dx_1 + dx_2)/2$ | $Ersy_2 = ys_2' - (dy_1 + dy_2)/2$ |
| $S_3$ | $Ersx_3 = xs_3' - (dx_1 + dx_2 + dx_3)/3$ | $Ersy_3 = ys_3' - (dy_1 + dy_2 + dy_3)/3$ |

Whether the systematic error $Ess_{i,k}$ exists within the first permissible range is determined (step S6O). The systematic error $Ess_k$ and the random error $Ers_k$ obtained by the error operating apparatus 56B are input to the error determination apparatus 57B. The error determination apparatus 57B firstly determines whether the systematic error $Ess_k$ exists within the first permissible range.

The first permissible range of the systematic error $Ess_{i,k}$ is the same as the permissible range As of the systematic error $Es_{i,j}$ shown in FIG. 10. When the systematic error $Ess_k$ is within the upper and lower limit, it is determined that the systematic error $Ess_k$ exists within the first permissible range. Namely, the determination at step S6O becomes "Yes". When it is determined that the systematic error $Ess_k$ has deviated from the first permissible range and the determination at step S6O becomes "No".

When the systematic error $Essx_k$ of the systematic error $Ess_k$ in the x direction and the systematic error $Essy_k$ in the y direction are within the upper and lower limit, respectively, it is determined that the systematic error $Ess_k$ exists within the first permissible range. Namely, the determination at step S6O becomes "Yes".

When it is determined that the systematic error $Ess_k$ does not exist within the first permissible range, namely, that the systematic error $Ess_k$ has deviated from the first permissible range, and the determination at step S6O becomes "No".

When the determination at step S6O is "No", the error determination apparatus 57B outputs the beam irradiation stop signal at step S18. The ion beam irradiation is stopped.

When the determination at step S6O is "Yes", it is determined whether the random error $Ers_{i,k}$ exists within the second permissible range (step S6P). The error determination apparatus 57B determines whether the random error $Ers_k$ exists within the second permissible range. The second permissible range of the random error $Ers_k$ is the same as the permissible range Ar of the random error $Er_{i,j}$ shown in FIG. 10.

When the random error $Ers_k$ is within the upper and lower limit, it is determined that the random error $Ers_k$ exists within the second permissible range. Namely, the determination at step S6P becomes "Yes". When it is determined that the random error $Ers_k$ has deviated from the second permissible range and the determination at step S6P becomes "No".

When the random error $Ersx_j$ of the random error $Ers_k$ in the x direction and the random error $Ersy_j$ in the y direction are within the upper and lower limit, respectively, it is determined that the random error $Ers_j$ exists within the second permissible range. Namely, the determination at step S6P is "Yes".

$Pmsx_k$ is a coordinate of the mean position $Pms_k$ of the actual irradiation position $Pas_k$ in the x direction and $Pmsy_k$ is a coordinate of the mean position $Pms_k$ of the actual irradiation position $Pas_k$ in the y direction.

When it is determined that the random error $Ers_k$ has deviated from the second permissible range and the determination at step S6P becomes "No". When the determination at step S6N is "No", steps S18 and S19 are executed.

After step S6P finishes, $s = s + 1$ is calculated and s becomes 2. Whether the second beam irradiation section $S_2$ in the irradiation spot $A_{1,12}$ is the last beam irradiation section in the irradiation spot $A_{1,12}$ is determined at step S6H. Three beam irradiation sections exist in the irradiation spot $A_{1,12}$, so that the determination at step S6H becomes "No" and each of the processes steps S6I to S6P for the beam irradiation section $S_2$ is repeated similarly to the beam irradiation section $S_1$.

Particularly, at step S6L, the beam position monitor 30 measures the actual irradiation position $Pas_{1,2}$ ($xs_{1,2}'$, $ys_{1,2}'$) of the ion beam irradiated to the target position $P_{1,12}$ ($x_{1,12}$, $y_{1,12}$) of the irradiation spot $A_{1,12}$ in the beam irradiation section $S_2$ in the irradiation spot $A_{1,12}$. At Step S6M, as a deviation $d_2$ between the target position $P_1$ and the actual irradiation position $Pas_2$, $dx_2$ (= $xs_2$ - $xs_2'$) and $dy_2$ (= $ys_2$ - $ys_2'$) are obtained. The information of the deviation $d_2$ is stored in the memory 60 of the scanning control apparatus 40. At step S6N for the beam irradiation section $S_2$, the systematic errors $Essx_2$ and $Essy_2$ shown in Table 3 are obtained and the random errors $Ersx_2$ and $Ersy_2$ shown in Table 4 are obtained. Each determination of the systematic error $Ess_2$ and the random error $Ers_2$ is performed at steps S6O and S6P.

In the next determination at step S6H, the beam irradiation section No. 3 in the irradiation spot $A_{1,12}$ is the last beam irradiation section in the irradiation spot $A_{1,12}$, so that the determination at step S6H becomes "Yes". Therefore, each of the processes steps S6A to S6E shown in FIG. 12 is executed. At step S6A, the actual irradiation position $Pa_{1,12}$ measured in the beam irradiation section No. 3 is input. Each process of steps S6B to S6E using the actual irradiation position $Pa_{1,12}$ of the irradiation spot $A_{1,12}$ is performed similarly to embodiment 1. Each determination at steps S6D and S6E is "Yes" and at step S7, the dose determination apparatus 53 determines whether the dose $R_{1,12}$ of the actual irradiation position $Pa_{1,12}$ measured by the dose monitor 31 and the target dose $R0_{1,12}$ of the irradiation spot $A_{1,12}$ coincide with each other. Further, when the measurement of the dose in the beam irradiation section $S_1$ in the irradiation spot $A_{1,12}$ is started by the dose monitor 31A at step S6I, the measurement of the dose $R_{1,12}$ in the actual irradiation position $Pa_{1,12}$ of the irradiation spot $A_{1,12}$ by the dose monitor 31 is started. The measurement of the dose $R_{1,12}$ by the dose monitor 31 is performed in each of the beam irradiation sections $S_1$, $S_2$, and No. 3 in the irradiation spot $A_{1,12}$. When the determination at step S7 becomes "Yes", the dose determination apparatus 53 outputs the beam irradiation stop signal (step S9A) and the irradiation of the ion beam to the irradiation spot $A_{1,12}$ is stopped by the control by the accelerator-and-transport-system control apparatus 39 (step S10). The next determination at step S11 becomes "No".

Each process of steps S13, S14, and S15 is executed. Each process from step S2 or step S4 are executed according to the determination results at step S15. At step S4, each bending electromagnetic force of the scanning magnets 28 and 29 is adjusted so that the irradiation position of the ion beam becomes the target position $P_{1,13}$ of the irradiation spot $A_{1,13}$. Thereafter, the process of step S5 is executed. The beam irradiation section S (refer to FIG. 13) is not set for the irradiation spot $A_{1,13}$ (the irradiation spot No. 3), so that the determination at step S6G becomes "No" similarly to the irradiation spots $A_{1,1}$ to $A_{1,11}$, and each process of steps S6A to S6E is executed. The determination at step S7 or S9 becomes "Yes" and steps S9A, S10, and S11 are executed. If the determination at step S6D or S6E becomes "No", steps S18 and S19 are executed.

Furthermore, each process of steps S13 to S15 is executed. Each process from step S2 or step S4 are executed according to the determination results at step S15. At step S4, each bending electromagnetic force of the scanning magnets 28 and 29 is adjusted so that the irradiation position of the ion beam becomes the target position $P_{1,14}$ of the irradiation spot $A_{1,14}$ (the irradiation spot No. 4). Thereafter, the process of step S5 is executed. In the irradiation spot $A_{1,14}$, two beam irradiation sections S are set (refer to FIG. 13). For the irradiation spot $A_{1,14}$, the determination at step S6G becomes "Yes", and because the beam irradiation section $S_3$ in the irradiation spot $A_{1,14}$ is not the last beam irradiation section in the irradiation spot $A_{1,14}$, the determination at step S6H becomes "No". Each process of steps S6I to S6P for the beam irradiation section $S_3$ is repeated similarly to the beam irradiation section $S_1$. When the determination at step S6O or S6P becomes "No", steps S18 and S19 are executed.

At step S6L, the beam position monitor 30 measures the actual irradiation position $Pas_{1,3}$ ($xs_{1,3}'$, $ys_{1,3}'$) of the ion beam irradiated to the target position $P_{1,14}$ ($x_{1,14}$, $y_{1,14}$) of the irradiation spot $A_{1,14}$ in the beam irradiation section $S_3$ in the irradiation spot $A_{1,14}$. At step S6M, as a deviation $d_3$ between the target position $P_{14}$ and the actual irradiation position $Pas_3$, $dx_3$ (= $xs_3$ - $xs_3'$) and $dy_3$ (= $ys_3$ - $ys_3'$) are obtained. The information of the deviation $d_3$ is stored in the memory 60 of the scanning control apparatus 40. At step S6N for the beam irradiation section $S_3$, the systematic errors $Essx_3$ and $Essy_3$ shown in Table 3 are obtained and the random errors $Ersx_3$ and $Ersy_3$ shown in Table 4 are obtained. Each determination of the systematic error $Ess_3$ and the random error $Ers_3$ is performed at steps S6O and S6P.

Next, the irradiation of the ion beam in the beam irradiation section No. 5 in the irradiation spot $A_{1,14}$ is performed. The determination at step S6H becomes "Yes" and each process of steps S6A to S6E, S7 to S11, and S13 is executed similarly to the beam irradiation section No. 3 in the irradiation spot $A_{1,14}$.

At step S14, j becomes 15 and the ion beam irradiation for each irradiation spot $A_{1,j}$ in the layer L from the irradiation spot $A_{1,15}$ afterward is performed successively. In the irradiation spot $A_{1,j}$ including a plurality of beam irradiation sections, each process of steps S6I to S6P is executed for the beam irradiation sections other than the last beam irradiation section and each process of steps S6A to S6E and S7 to S10 is executed for the last beam irradiation section. When the ion beam irradiation to all the irradiation spots $A_{1,j}$ in the layer $L_1$ finishes, the determination at step S11 becomes "Yes" and at step S12, information of all the deviations $D_j$ and all the deviations $d_k$ for the layer $L_1$ is deleted.

The determination at step S16 becomes "No" and the ion beam is irradiated successively for the target position $Pi,j$ of each irradiation spot $Ai,j$ in the next layer $L_2$. When the irradiation of the ion beam to the target position $P_{i,j}$ of all the irradiation spots $A_{i,j}$ in the last layer $L_m$ finishes and the determination at step S16 becomes "Yes", the treatment by the ion beam irradiation to the patient 34 finishes.

Further, the permissible range As used for the determination of the systematic error Es at steps S6D and S6O of the present example and the permissible range Ar used for the determination of the random error Er at steps S6E and S6N are wider than the permissible range As used for the determination when the determination count is h+1 times or more and the permissible range As and the permissible range Ar used at steps S6E and S6P in the case of the determination count of h times or smaller, similarly to those permissible ranges used in embodiment 1.

The present example can obtain each effect generated in embodiment 1. In the present example, the systematic error Ess and the random error Ers are obtained also for the beam irradiation section and whether each of them exists within the permissible ranges is determined, so that the frequency of the determination of the systematic error and the random error increases and the safety increases more.

[Embodiment 2]

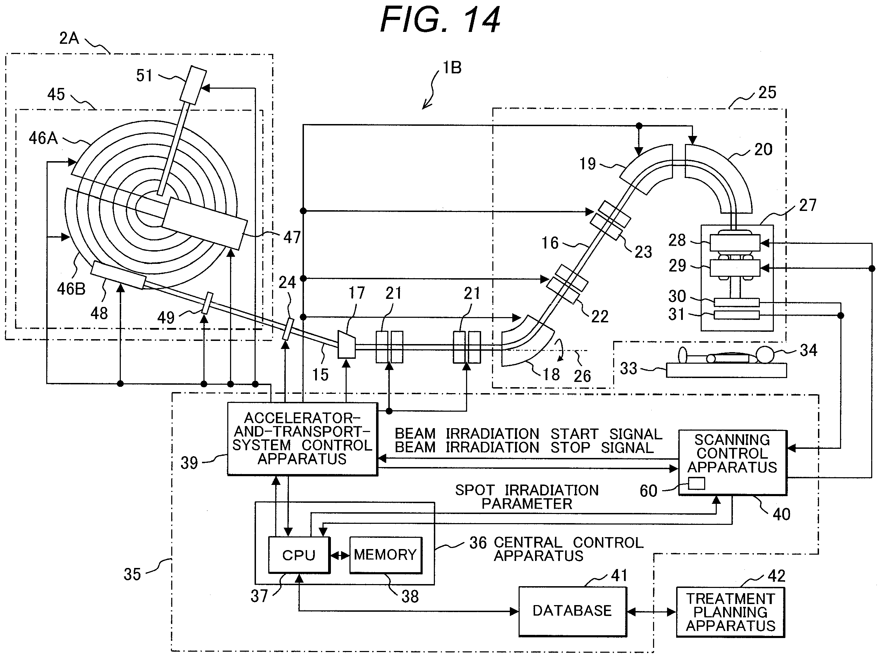

A charged particle beam irradiation system according to embodiment 2 which is other preferred embodiment of the present invention will be explained by referring to FIG. 14.

The charged particle beam irradiation system according to embodiment 1 and the example uses a synchrotron accelerator as an accelerator for accelerating the ion beam. By contrast, the charged particle beam irradiation system 1B of the present embodiment uses a cyclotron accelerator 45 as the accelerator. The charged particle beam irradiation system 1B includes a charged particle generating apparatus 2A, the beam transport system 15, the rotating gantry 25, the irradiation nozzle 27, and the control system 35. The beam transport system 15, the rotating gantry 25, and the irradiation nozzle 27 used in the charged particle beam irradiation system 1B have the same structures as those used in the charged particle beam irradiation system 1 of embodiment 1. The charged particle generating apparatus 2A is different from the charged particle generating apparatus 2 of the charged particle beam irradiation system 1 and includes an ion source 51 and the cyclotron accelerator 45. The charged particle generating apparatus 2 also includes the ion source 51 connected to the linear accelerator 14. The charged particle generating apparatus 2A does not include the linear accelerator 14. The cyclotron accelerator 45 includes a circular vacuum vessel (not shown), bending magnets 46A and 46B, a radiofrequency acceleration apparatus 47, and an extraction deflector 48. The vacuum duct connected to the ion source 51 extends up to a central position of the vacuum vessel of the cyclotron accelerator 45 and is communicated with the vacuum vessel. The bending magnets 46A and 46B are semicircular, are disposed so that the straight portions are opposite to each other, and cover a top and bottom of the vacuum vessel.

The extraction deflector 48 installed at the ion beam irradiation outlet of the vacuum vessel is connected to the beam path 16 of the beam transport system 15. A degrader 49 made of metal is attached to the beam path 16 between the extraction deflector 48 and the shutter 24. The degrader 49 has a function for adjusting the energy of the ion beam extracted from the cyclotron accelerator 45 and includes a plurality of metallic plates (not shown) different in thickness. These metallic plates can move in the direction perpendicular to the beam path 16. One or the a plurality of plates different in thickness are inserted into the beam path 16 so as to cross the beam path 16, thus the attenuation rate of the energy of the ion beam is controlled. As a result, the energy irradiated to the target volume of the patient 34 can be changed and each layer existing in the depth direction of the target volume can be irradiated with the ion beam.

In the charged particle beam irradiation system 1B, the scanning control apparatus 40 of the control system 35 has the same structure as that of the scanning control apparatus 40 of the charged particle beam irradiation system 1 and the central control apparatus 36 also has the substantially same function as that of the central control apparatus 36 of the charged particle beam irradiation system 1. Partial control subjects controlled by the accelerator-and-transport-system control apparatus 39 of the charged particle beam irradiation system 1B are different from those controlled by the accelerator-and-transport-system control apparatus 39 of the charged particle beam irradiation system 1 because of the use of the cyclotron accelerator 45. The accelerator-and-transport-system control apparatus 39 of the charged particle beam irradiation system 1B controls the shutter 24 of the beam transport system 15, the bending magnets 17, 18, 19, and 20 of the beam transport system 15, and the quadrupole magnets 21, 22, and 23 similarly to the accelerator-and-transport-system control apparatus 39 of the charged particle beam irradiation system 1 and other than these, controls also the ion source 51, the bending magnets 46A and 46B, the radiofrequency acceleration apparatus 47, the extraction deflector 48, and the degrader 49.

A method of irradiating charged particle beam using the charged particle beam irradiation system 1B will be explained below. In this method of irradiating charged particle beam, each process of steps S1 to S19 shown in FIGs. 2A and 2B is executed. At step S2, the ion source 51 is started, though the linear accelerator is not started. Step S6 includes steps S6A to S6E shown in FIG. 3.

Each process of steps S1 to S3 and S5 is executed by the accelerator-and-transport-system control apparatus 39. At step S1, by the accelerator-and-transport-system control apparatus 39, the shutter 24 is opened and each magnet installed on the beam transport system 15 is excited similarly to embodiment 1. At step S2, the ion source 51 is started and the proton ions generated in the ion source 51 are injected to the center of the vacuum vessel of the cyclotron accelerator 45 through the vacuum duct. The bending magnets 46A and 46B are excited already. At step S3, the proton ions injected into the vacuum vessel are accelerated by the radiofrequency acceleration apparatus 47 and the proton ion beam having large energy is generated.

At step S4, the irradiation position control apparatus 52 adjusts the respective bending electromagnetic forces of the scanning magnets 28 and 29 so that the target position $P_{1,1}$ of the irradiation spot $A_{1,1}$ in the deepest layer $L_1$ of the target volume is irradiated with the ion beam. Thereafter, at step S3, the ion beam accelerated by the cyclotron accelerator 45 is extracted from the extraction deflector 48 to the beam path 16 (step S5). The tumor volume of the patient 34 on the treatment bed 33 is irradiated with the ion beam from the irradiation nozzle 27. Thereafter, each step of steps S6A to S6E, S7 to S9, S9A, S10, and S11 is executed similarly to embodiment 1.

If the determination at step S6D or S6E is "No", the error determination apparatus 57 outputs the beam irradiation stop signal (step S18). The accelerator-and-transport-system control apparatus 39 inputting the beam irradiation stop signal stops the ion source 51 and makes the shutter 24 insert into the beam path 16. By doing this, the irradiation of the ion beam to the target volume is stopped (step S19). The irradiation of the ion beam to the target volume is stopped also by either of the stop of the ion source 51 and the insertion of the shutter 24. When each determination at steps S6D and S6E is "Yes", each step of steps S7 to S9, S9A, S10, and S11 is executed.

The target positions $P_{1,2}$, $P_{1,3}$, ..., $P_{1,m}$ of the irradiation spot $A_{1,2}$ and after the irradiation spot $A_{1,1}$, the respective target positions $P_{1,j}$ of all the irradiation spots $A_{1,j}$ (j = 2, 3, ..., n) in the layer $L_1$ is irradiated successively with the ion beam. For the irradiation spots $A_{1,j}$ (j = 2, 3, ..., n), each step of steps S6A to S6E, S7 to S9, S9A, S10, and S11 is executed successively. When the determination at step S11 becomes "Yes", the irradiation of the ion beam to the layer $L_1$ finishes and next, all the irradiation spots $A_{2,j}$ in the layer $L_2$ in the shallower position than the layer $L_1$ are irradiated successively with the ion beam.

The energy of the ion beam irradiated to the layer $L_2$ must be lower than that of the ion beam irradiated to the layer $L_1$. Therefore, the accelerator-and-transport-system control apparatus 39 scans the degrader 49 and inserts a thinnest metallic plate perpendicularly to the beam path 16. The energy of the ion beam extracted from the cyclotron accelerator 45 is attenuated by the thinnest metallic plate of the degrader 49, thereby generating an ion beam having energy forming a Bragg peak in the layer $L_2$. The irradiation spot $A_{2,j}$ in the layer $L_2$ of the target volume is irradiated successively with this ion beam. At the time of the irradiation of the ion beam to the shallower layer of the target volume, the thickness of the metallic plate of the degrader 49 for inserting it into the beam path 16 across the beam path 16 is increased more. The increase in the thickness of the plate can be attained also in combination with a plurality of plates different in thickness instead of only one plate in the degrader 49.

The present embodiment can obtain each effect generated in embodiment 1.

Even when the cyclotron accelerator 45 is used, by the scanning control apparatus 40, the process of step S6 may be replaced with the process of step S6Q shown in FIG. 12 as in the example.

The charged particle beam irradiation systems 1, 1A, and 1B used in embodiments 1 to 2 may accelerate a carbon ion beam instead of the proton ion beam and the target volume is irradiated with the accelerated carbon ion beam.

Features, components and specific details of the structures of the above-described embodiments may be exchanged or combined to form further embodiments optimized for the respective application. As far as those modifications are apparent for an expert skilled in the art they shall be disclosed implicitly by the above description without specifying explicitly every possible combination.

[REFERENCE SIGNS LIST]

1, 1A, 1B : charged particle beam irradiation system, 2, 2A : charged particle generating apparatus, 3 : synchrotron accelerator, 6, 17, 18-20, 46A,46B : bending magnet, 8 : acceleration apparatus (acceleration cavity), 9 : radiofrequency application apparatus, 10 : extraction radiofrequency electrode, 12 : open/close switch, 14 : linear accelerator, 15 : beam transport system, 16 : beam path, 24 : shutter, 25 : rotating gantry, 27 : irradiation nozzle, 28, 29 : scanning magnet, 30 : beam position monitor, 31 : dose monitor, 35 : control system, 36 : central control apparatus, 39 : accelerator-and-transport-system control apparatus, 40 : scanning control apparatus, 42 : treatment planning apparatus, 47 : radiofrequency acceleration apparatus, 49 : degrader, 51 : ion source, 52 : irradiation position control apparatus, 53, 59 : dose determination apparatus, 54 : layer determination apparatus, 55, 55A : beam position monitoring apparatus, 56, 56A, 56B : error operating apparatus, 57, 57A, 57B : error determination apparatus, 58 : beam irradiation section determination apparatus, 60 : memory.

**Claims**

**1.** A beam position monitoring apparatus (55) comprising:

an error operating apparatus (56) adapted to obtain a deviation between a target position in a beam irradiation subject which is irradiated with a charged particle beam from an irradiation nozzle (27) and an actual irradiation position which is irradiated with said charged particle beam in said beam irradiation subject in correspondence to said target position, said actual irradiation position being measured by a beam position monitor (30) installed in said irradiation nozzle, **characterized in that** the apparatus is further adapted to obtain individually a systematic error and a random error for said actual irradiation position based on said deviation; and
the beam position monitoring apparatus further comprising
an error determination apparatus (57) adapted to execute a first determination for determining whether said systematic error exists within a first permissible range of said systematic error and a second determination for determining whether said random error exists within a second permissible range of said random error;
wherein the systematic error is obtained based on a sum of said deviations for multiple irradiation positions according to formulas (1) and (2), and the random error is obtained based on a difference of said actual irradiation position and the systematic error according to formulas (1), (2) and (3), when said target position in a certain layer among plurality of layers formed in a direction of a central axis of said irradiation nozzle (27) is $P_j$ (j=1,...,n), said actual irradiation position corresponding to said target position is $Pa_j$, said deviation is $D_j$, said systematic error is $Es_j$, said random error is $Er_j$, and

$$D_j = P_j - Pa_j \quad ...(1)$$

$$Es_j = \frac{\sum_{k=1}^{j} D_k}{j} \quad ...(2)$$

$$Er_j = P_j - Pa_j - \frac{\sum_{k=1}^{j} D_k}{j} \quad ...(3).$$

2. The beam position monitoring apparatus (55) according to claim 1, comprising:
said error determination apparatus (57) adapted to output a beam irradiation stop signal when it is determined in said first determination that said systematic error does not exist within said first permissible range of said systematic error or when it is determined in said second determination that said random error does not exist within said second permissible range of said random error.

3. A charged particle beam irradiation system comprising:

an accelerator adapted to irradiate a charged particle beam; an irradiation nozzle (27) adapted to output said charged particle beam extracted from said accelerator, and including a charged particle beam scanning apparatus (28, 29); an irradiation position control apparatus (52) adapted to control said charged particle beam scanning apparatus (28, 29) and to adjust an irradiation position of said charged particle beam to said target position based on a target position in a beam irradiation subject which is irradiated with said charged particle beam; a beam position monitor (30) adapted to measure an actual irradiation position of said charged particle beam, and installed in said irradiation nozzle (27); and a beam position monitoring apparatus (55),
wherein said beam position monitoring apparatus is said beam position monitoring apparatus (55) according to claim 1.

4. The charged particle beam irradiation system according to claim 3, comprising:

said error determination apparatus (57) adapted to output a beam irradiation stop signal when it is determined that a systematic error does not exist within a first permissible range in said first determination or when it is determined that a random error does not exist within a second permissible range in said second determination.

5. The charged particle beam irradiation system according to at least one of claims 3 to 4, comprising:
a dose monitor (31) installed in said irradiation nozzle; and a dose determination apparatus (53) adapted to determine whether a dose in an actual irradiation position measured by said dose monitor (31) coincides with a target dose when said systematic error exists within said first permissible range and said random error exists within said second permissible range.

6. The charged particle beam irradiation system according to at least one of claims 3 to 4, comprising:

a beam transport system (15) adapted to introduce said charged particle beam extracted from said accelerator to said irradiation nozzle (27); a shutter (24) installed in a beam path of said beam transport system, and an accelerator-and-transport-system control apparatus (39) adapted to control excitation currents of a plurality of magnets installed on said accelerator and said beam transport system,
wherein said accelerator-and-transport-system control apparatus (39) is adapted to insert said shutter (24) into said beam path and to block said beam path based on said beam irradiation stop signal from said error determination apparatus (57).

7. The charged particle beam irradiation system according to at least one of claims 3 to 6,
wherein said accelerator is a synchrotron accelerator;
wherein said synchrotron accelerator includes an acceleration apparatus (8), and a radiofrequency application apparatus (9) adapted to apply radiofrequency to said charged particle beam in said synchrotron accelerator when said charged particle beam is extracted from said synchrotron accelerator;
wherein said charged particle beam irradiation system includes a beam transport system adapted to introduce said charged particle beam extracted from said synchrotron accelerator to said irradiation nozzle (27); and an accelerator-and-transport-system control apparatus (39) adapted to control excitation currents of a plurality of magnets installed individually on said synchrotron accelerator and said beam transport system, and to control said acceleration apparatus, and said accelerator-and-transport-system control apparatus (39) being adapted to stop said application of said radiofrequency to said charged particle beam by said radiofrequency application apparatus (9) based on said beam irradiation stop signal from said error determination apparatus (57).

## Patentansprüche

1. Strahlpositionsüberwachungsvorrichtung (55), die Folgendes umfasst:

eine Fehlerbehandlungsvorrichtung (56), die dafür ausgelegt ist, eine Abweichung zwischen einer Zielposition in einer mit einem Strahl bestrahlten Person, die mit einem Strahl geladener Partikel aus einer Bestrahlungsdüse (27) bestrahlt wird, und einer tatsächlichen Bestrahlungsposition, die mit dem Strahl geladener Partikel in der mit einem Strahl bestrahlten Person in Verbindung mit der Zielposition bestrahlt wird, zu erhalten, wobei die tatsächliche Bestrahlungsposition durch eine Strahlpositionsüberwachungseinrichtung (30), die in der Bestrahlungsdüse installiert ist, gemessen wird,
**dadurch gekennzeichnet, dass** die Vorrichtung ferner dafür ausgelegt ist, individuell einen systematischen Fehler und einen zufälligen Fehler für die tatsächliche Bestrahlungsposition auf der Grundlage der Abweichung zu erhalten, und
die Strahlpositionsüberwachungsvorrichtung ferner Folgendes umfasst:

eine Fehlerbestimmungsvorrichtung (57), die dafür ausgelegt ist, Folgendes auszuführen: eine erste Bestimmung zum Bestimmen, ob der systematische Fehler in einem ersten zulässigen Bereich des systematischen Fehlers vorliegt, und eine zweite Bestimmung zum Bestimmen, ob der zufällige Fehler in einem zweiten zulässigen Bereich des zufälligen Fehlers vorliegt;
wobei der systematische Fehler auf der Grundlage einer Summe der Abweichungen für mehrere Bestrahlungspositionen entsprechend den Formeln (1) und (2) erhalten wird und der zufällige Fehler auf der Grundlage einer Differenz der tatsächlichen Bestrahlungsposition und des systematischen Fehlers entsprechend den Formeln (1), (2) und (3) erhalten wird, dann, wenn die Zielposition in einer bestimmten Schicht unter den mehreren Schichten, die in einer Richtung einer zentralen Achse der Bestrahlungsdüse (27) gebildet sind, $P_j$ ist ($j = 1,...,n$), die tatsächliche Bestrahlungsposition, die der Zielposition entspricht, $Pa_j$ ist, die Abweichung $D_j$ ist, der systematische Fehler $Es_j$ ist, der zufällige Fehler $Er_j$ ist, und

$$D_j = P_j - Pa_j \qquad ...(1)$$

$$Es_j = \frac{\sum_{k=1}^{j} D_k}{j} \qquad ...(2)$$

$$Er_j = P_j - Pa_j - \frac{\sum_{k=1}^{j} D_k}{j} \qquad ...(3).$$

**2.** Strahlpositionsüberwachungsvorrichtung (55) nach Anspruch 1, die Folgendes umfasst:
die Fehlerbestimmungsvorrichtung (57), die dafür ausgelegt ist, ein Strahlabstrahlungsstoppsignal auszugeben, wenn in der ersten Bestimmung bestimmt wird, dass der systematische Fehler nicht in dem ersten zulässigen Bereich des systematischen Fehlers vorliegt, oder wenn in der zweiten Bestimmung bestimmt wird, dass der zufällige Fehler nicht in dem zweiten zulässigen Bereich des zufälligen Fehlers vorliegt.

**3.** Bestrahlungssystem mit einem Strahl geladener Partikel, das Folgendes umfasst:

eine Beschleunigungseinrichtung, die dafür ausgelegt ist, einen Strahl geladener Partikel abzustrahlen; eine Bestrahlungsdüse (27), die dafür ausgelegt ist, den von der Beschleunigungseinrichtung extrahierten Strahl geladener Partikel auszugeben, und die eine Abtastvorrichtung (28, 29) für einen Strahl geladener Partikel enthält; eine Bestrahlungspositionssteuervorrichtung (52), die dafür ausgelegt ist, die Abtastvorrichtung (28, 29) für einen Strahl geladener Partikel zu steuern und eine Bestrahlungsposition des Strahls geladener Partikel auf der Grundlage einer Zielposition in einer mit einem Strahl bestrahlten Person, die mit dem Strahl bestrahlter Partikel bestrahlt wird, auf eine Zielposition einzustellen; eine Strahlpositionsüberwachungseinrichtung (30), die dafür ausgelegt ist, eine tatsächliche Bestrahlungsposition des Strahls geladener Partikel zu messen, und die in der Bestrahlungsdüse (27) installiert ist; und eine Strahlpositionsüberwachungsvorrichtung (55), wobei die Strahlpositionsüberwachungsvorrichtung die Strahlpositionsüberwachungsvorrichtung (55) nach Anspruch 1 ist.

**4.** Bestrahlungssystem mit einem Strahl geladener Partikel nach Anspruch 3, das Folgendes umfasst:
die Fehlerbestimmungsvorrichtung (57), die dafür ausgelegt ist, ein Strahlabstrahlungsstoppsignal auszugeben, wenn in der ersten Bestimmung bestimmt wird, dass ein systematischer Fehler nicht in einem ersten zulässigen Bereich vorliegt, oder wenn in der zweiten Bestimmung bestimmt wird, dass ein zufälliger Fehler nicht in einem zweiten zulässigen Bereich vorliegt.

**5.** Bestrahlungssystem mit einem Strahl geladener Partikel nach mindestens einem der Ansprüche 3 oder 4, das Folgendes umfasst:
eine Dosisüberwachungseinrichtung (31), die in der Bestrahlungsdüse installiert ist; und eine Dosisbestimmungsvorrichtung (53), die dafür ausgelegt ist, zu bestimmen, ob eine von der Dosisüberwachungseinrichtung (31) gemessene Dosis in einer tatsächlichen Bestrahlungsposition mit einer Zieldosis übereinstimmt, wenn der systematische Fehler in dem ersten zulässigen Bereich vorliegt und der zufällige Fehler in dem zweiten zulässigen Bereich vorliegt.

**6.** Bestrahlungssystem mit einem Strahl geladener Partikel nach mindestens einem der Ansprüche 3 oder 4, das Folgendes umfasst:

ein Strahltransportsystem (15), das dafür ausgelegt ist, den aus der Beschleunigungseinrichtung extrahierten Strahl geladener Partikel in die Bestrahlungsdüse (27) einzuleiten; einen Verschluss (24), der in einem Strahlpfad des Strahltransportsystems installiert ist, und eine Beschleunigungs-und-Transport-Systemsteuervorrichtung (39), die dafür ausgelegt ist, Erregungsströme von mehreren Magneten, die auf der Beschleunigungseinrichtung und dem Strahltransportsystem installiert sind, zu steuern, wobei die Beschleunigungs-und-Transport-Systemsteuervorrichtung (39) dafür ausgelegt ist, den Verschluss (24) in den Strahlpfad einzusetzen und den Strahlpfad auf der Grundlage des Strahlabstrahlungsstoppsignals von der Fehlerbestimmungsvorrichtung (57) zu versperren.

**7.** Bestrahlungssystem mit einem Strahl geladener Partikel nach mindestens einem der Ansprüche 3 bis 6, wobei die Beschleunigungseinrichtung eine Synchrotronbeschleunigungseinrichtung ist;
die Synchrotronbeschleunigungseinrichtung Folgendes umfasst: eine Beschleunigungsvorrichtung (8) und eine Hochfrequenzanwendungsvorrichtung (9), die dafür ausgelegt ist, eine Hochfrequenz auf den Strahl geladener Partikel in der Synchrotronbeschleunigungseinrichtung anzuwenden, dann, wenn der Strahl geladener Partikel aus der Synchrotronbeschleunigungseinrichtung extrahiert wird;

wobei das Bestrahlungssystem mit einem Strahl geladener Partikel Folgendes umfasst: ein Strahltransportsystem, das dafür ausgelegt ist, den aus der Beschleunigungseinrichtung extrahierten Strahl geladener Partikel in die Bestrahlungsdüse (27) einzuleiten; und eine Beschleunigungs-und-Transport-Systemsteuervorrichtung (39), die dafür ausgelegt ist, Erregungsströme von mehreren Magneten, die auf der Beschleunigungseinrichtung und dem Strahltransportsystem installiert sind, zu steuern und die Beschleunigungsvorrichtung zu steuern, und die Beschleunigungs-und-Transport-Systemsteuervorrichtung (39) dafür ausgelegt ist, die Anwendung der Hochfrequenz auf den Strahl geladener Partikel durch die Hochfrequenzanwendungsvorrichtung (9) auf der Grundlage des Strahlabstrahlungsstoppsignals von der Fehlerbestimmungsvorrichtung (57) zu stoppen.

**Revendications**

1. Appareil de surveillance de position de faisceau (55) comprenant :

   un appareil d'exploitation d'erreur (56) adapté à obtenir une déviation entre une position cible dans un sujet d'irradiation par faisceau qui est irradié avec un faisceau de particules chargées à partir d'une buse d'irradiation (27) et une position d'irradiation réelle qui est irradiée avec ledit faisceau de particules chargées dans ledit sujet d'irradiation par faisceau en correspondance avec ladite position cible, ladite position d'irradiation réelle étant mesurée par un moniteur de position de faisceau (30) installé dans ladite buse d'irradiation, **caractérisé en ce que** l'appareil est en outre adapté à obtenir individuellement une erreur systématique et une erreur aléatoire pour, ladite position d'irradiation réelle sur la base de ladite déviation ; et que l'appareil de surveillance de position de faisceau comprend en outre

   un appareil de détermination d'erreur (57) adapté à exécuter une première détermination pour déterminer si ladite erreur systématique existe dans une première plage admissible de ladite erreur systématique et une seconde détermination pour déterminer si ladite erreur aléatoire existe dans une seconde plage admissible de ladite erreur aléatoire ;

   dans lequel l'erreur systématique est obtenue sur la base d'une somme desdites déviations pour de multiples positions d'irradiation selon les formules (1) et (2), l'erreur aléatoire est obtenue sur la base d'une différence de ladite position d'irradiation réelle et de l'erreur systématique selon les formules (1), (2) et (3), lorsque ladite position cible dans une certaine couche parmi la pluralité de couches formées dans une direction d'un axe central de ladite buse d'irradiation (27) est $P_j$ (j = 1, ..., n), ladite position d'irradiation réelle correspondant à ladite position cible est $Pa_j$, ladite déviation est $D_j$, ladite erreur systématique est $Es_j$, ladite erreur aléatoire est $Er_j$, et

$$D_j = P_j - Pa_j \quad ...(1)$$

$$Es_j = \frac{\sum_{k=1}^{j} D_k}{j} \quad ...(2)$$

$$Er_j = P_j - Pa_j - \frac{\sum_{k=1}^{j} D_k}{j} \quad ...(3).$$

2. Appareil de surveillance de position de faisceau (55) selon la revendication 1, comprenant : ledit appareil de détermination d'erreur (57) adapté pour délivrer un signal d'arrêt d'irradiation de faisceau lorsqu'il est déterminé dans ladite première détermination que ladite erreur systématique n'existe pas dans ladite première plage admissible de ladite erreur systématique ou lorsqu'il est déterminé dans ladite seconde détermination que ladite erreur aléatoire n'existe pas dans ladite seconde plage admissible de ladite erreur aléatoire.

3. Système d'irradiation par faisceau de particules chargées comprenant :

   un accélérateur adapté à irradier un faisceau de particules chargées ;

une buse d'irradiation (27) adaptée à délivrer ledit faisceau de particules chargées extrait dudit accélérateur, et incluant un appareil de balayage de faisceau de particules chargées (28, 29) ; un appareil de commande de position d'irradiation (52) adapté à commander ledit appareil de balayage de faisceau de particules chargées (28, 29) et à ajuster une position d'irradiation dudit faisceau de particules chargées à ladite position cible sur la base d'une position cible dans un sujet d'irradiation par faisceau qui est irradié avec ledit faisceau de particules chargées; un moniteur de position de faisceau (30) adapté à mesurer une position d'irradiation réelle dudit faisceau de particules chargées et installé dans ladite buse d'irradiation (27) ; et un appareil de surveillance de position de faisceau (55),

dans lequel ledit appareil de surveillance de position de faisceau est ledit appareil de surveillance de position de faisceau (55) selon la revendication 1.

4. Système d'irradiation de faisceau de particules chargées selon la revendication 3, comprenant :
ledit appareil de détermination d'erreur (57) adapté à délivrer un signal d'arrêt d'irradiation de faisceau lorsqu'il est déterminé qu'une erreur systématique n'existe pas dans une première plage admissible dans ladite première détermination ou lorsqu'il est déterminé qu'une erreur aléatoire n'existe pas dans une seconde plage admissible dans ladite seconde détermination.

5. Système d'irradiation de faisceau de particules chargées selon au moins l'une des revendications 3 à 4, comprenant :
un moniteur de dose (31) installé dans ladite buse d'irradiation ; et un appareil de détermination de dose (53) adapté à déterminer si une dose dans une position d'irradiation réelle mesurée par ledit moniteur de dose (31) coïncide avec une dose cible lorsque ladite erreur systématique existe dans ladite première plage admissible et ladite erreur aléatoire existe dans ladite seconde plage admissible.

6. Système d'irradiation de faisceau de particules chargées selon au moins l'une des revendications 3 à 4, comprenant :

un système de transport de faisceau (15) adapté à introduire ledit faisceau de particules chargées extrait dudit accélérateur vers ladite buse d'irradiation (27) ; un obturateur (24) installé dans un trajet de faisceau dudit système de transport de faisceau, et un appareil de commande d'accélérateur et de système de transport (39) adapté à commander des courants d'excitation d'une pluralité d'aimants installés sur ledit accélérateur et ledit système de transport de faisceau,

dans lequel ledit appareil de commande d'accélérateur et de système de transport (39) est adapté à insérer ledit obturateur (24) dans ledit trajet de faisceau et à bloquer ledit trajet de faisceau sur la base dudit signal d'arrêt d'irradiation de faisceau provenant dudit appareil de détermination d'erreur (57).

7. Système d'irradiation par faisceau de particules chargées selon au moins l'une des revendications 3 à 6,
dans lequel ledit accélérateur est un accélérateur synchrotron ;
dans lequel ledit accélérateur synchrotron inclut un appareil d'accélération (8) et un appareil d'application de radiofréquence (9) adapté à appliquer une radiofréquence audit faisceau de particules chargées dans ledit accélérateur synchrotron lorsque ledit faisceau de particules chargées est extrait dudit accélérateur synchrotron ;
dans lequel ledit système d'irradiation de faisceau de particules chargées inclut un système de transport de faisceau adapté à introduire ledit faisceau de particules chargées extrait dudit accélérateur synchrotron vers ladite buse d'irradiation (27) ; et un appareil de commande d'accélérateur et de système de transport (39) adapté à commander des courants d'excitation d'une pluralité d'aimants installés individuellement sur ledit accélérateur synchrotron et ledit système de transport de faisceau et à commander ledit appareil d'accélération, et
ledit appareil de commande d'accélérateur et de système de transport (39) étant adapté à arrêter ladite application de ladite radiofréquence audit faisceau de particules chargées par ledit appareil d'application de radiofréquence (9) sur la base dudit signal d'arrêt d'irradiation de faisceau provenant dudit appareil de détermination d'erreur (57).

FIG. 1

TREATMENT PLANNING APPARATUS 42

DATABASE 41

CENTRAL CONTROL APPARATUS 36

CPU 37

MEMORY 38

SCANNING CONTROL APPARATUS 40

60

BEAM IRRADIATION START SIGNAL
BEAM IRRADIATION STOP SIGNAL

SPOT IRRADIATION PARAMETER

ACCELERATOR-AND-TRANSPORT-SYSTEM CONTROL APPARATUS 39

ACCELERATOR PARAMETER

35

33

34

27
20
25
28
29
30
31
23
19
22
16
18 26
21
21
17
15
24
2
3
5
14
6
7
9
8
4
13
11 12 10

1

# FIG. 2A

```
              ┌──────────┐
              │  START   │
              └──────────┘
                   │
                   ▼                    ⌇ S1
        ┌───────────────────────────┐
        │ CONTROL MAGNET OF BEAM    │
        │   TRANSPORT SYSTEM        │
        └───────────────────────────┘
                   │
                   ▼                    ⌇ S2
        ┌───────────────────────────┐
        │   START UP LINEAR          │
        │    ACCELERATOR             │
        └───────────────────────────┘
                   │
                   ▼                    ⌇ S3
        ┌───────────────────────────┐
        │  ACCELERATE ION BEAM       │
        │    IN ACCELERATOR          │
        └───────────────────────────┘
                   │
                   ▼                    ⌇ S4
        ┌ ─ ─ ─ ─ ─ ─ ─ ─ ─ ─ ─ ─ ─ ┐
        │ CONTROL SCANNING MAGNET   │
        │   AND SET ION BEAM        │        52
        │ IRRADIATION POSITION      │
        │ (SPOT POSITION) TO P_{i,j}│
        └ ─ ─ ─ ─ ─ ─ ─ ─ ─ ─ ─ ─ ─ ┘
                   │
                   ▼                    ⌇ S5
        ┌───────────────────────────┐
        │   EXTRACTE ION BEAM        │
        │   FROM ACCELERATOR         │
        └───────────────────────────┘
                   │
                   ▼
```

DETERMINE SYSTEMATIC ERROR AND RANDOM ERROR  ⌇ S6

EITHER ERROR IS OUTSIDE PERMISSIBLE VALUE

BOTH ERRORS ARE WITHIN PERMISSIBLE VALUE

⌇ S18
OUTPUT BEAM IRRADIATION STOP SIGNAL

⌇ S19
STOP EXTRACTION OF ION BEAM

(D) (C) (B) (A)

# FIG. 2B

## FIG. 3

```
                    ┌─────────────┐
                    │  STEP  S5   │
                    └─────────────┘
                           │
        ┌ ─ ─ ─ ─ ─ ─ ─ ─ ─│─ ─ ─ ─ ─ ─ ─ ─ ─ ─ ─ ┐
        ┆                   ▼            ⟋ S6A      ┆
        ┆  ┌──────────────────────────────────┐   ┆
        ┆  │ INPUT ACTUAL IRRADIATION POSITION │   ┆ ⟋ 56
        ┆  │  Pa_{i,j} OF IRRADIATION SPOT     │   ┆
        ┆  └──────────────────────────────────┘   ┆
        ┆                   │          ⟋ S6B       ┆
        ┆                   ▼                      ┆
        ┆  ┌──────────────────────────────────┐   ┆
        ┆  │ CALCULATE DEVIATION D_j BETWEEN   │   ┆
        ┆  │ TARGET POSITION P_{i,j} OF        │   ┆
        ┆  │ IRRADIATION SPOT AND ACTUAL       │   ┆
        ┆  │ IRRADIATION POSITION Pa_{i,j}     │   ┆
        ┆  └──────────────────────────────────┘   ┆
        ┆                   │          ⟋ S6C       ┆
        ┆                   ▼                      ┆
        ┆  ┌──────────────────────────────────┐   ┆ ⟋ 55
        ┆  │ CALCULATE SYSTEMATIC ERROR Es_{i,j}│  ┆
        ┆  │  AND RANDOM ERROR Er_{i,j}        │   ┆
        ┆  └──────────────────────────────────┘   ┆
        └ ─ ─ ─ ─ ─ ─ ─ ─ ─ │ ─ ─ ─ ─ ─ ─ ─ ─ ─ ─ ┘
```

- S6
- S6D: IS SYSTEMATIC ERROR $Es_{i,j}$ WITHIN FIRST PERMISSIBLE RANGE? — No / Yes
- S6E: IS RANDOM ERROR $Er_{i,j}$ WITHIN SECOND PERMISSIBLE RANGE? — No / Yes
- 57
- S18: OUTPUT BEAM IRRADIATION STOP SIGNAL
- S19: STOP ION BEAM IRRADIATION
- STEP S7

## FIG. 4

UPPER LIMIT VALUE OF RANDOM ERROR
PERMISSIBLE RANGE Ar

UPPER LIMIT VALUE OF SYSTEMATIC
ERROR PERMISSIBLE RANGE As

DEVIATION OF SPOT POSITION

MEAN VALUE OF SPOT
POSITION DEVIATION

SYSTEMATIC ERROR

RANDOM ERROR

LOWER LIMIT VALUE OF RANDOM
ERROR PERMISSIBLE RANGE Ar

NUMBER OF DETERMINATIONS : h TIMES

LOWER LIMIT VALUE OF SYSTEMATIC
ERROR PERMISSIBLE RANGE As

NUMBER OF DETERMINATIONS IN ONE LAYER

## FIG. 5

TARGET VOLUME

$A_{i,1}$ CENTER $P_{i,1}$ $(x_{i,1} \cdot y_{i,1})$

$A_{1,1}$ CENTER $P_{1,1}$ $(x_{1,1} \cdot y_{1,1})$

A CENTER $P_{1,2}$ $(x_{1,2} \cdot y_{1,2})$

$A_{1,j}$ CENTER $P_{1,j}$ $(x_{1,j} \cdot y_{1,j})$

$L_1$ $(E_1)$

$L_2$ $(E_2)$

$L_3$ $(E_3)$

$A_{i,j}$ CENTER $P_{i,j}$ $(x_{i,j} \cdot y_{i,j})$

$L_i$ $(E_i)$

$L_m$ $(E_m)$

50

IRRADIATION ENERGY $E_i$ OF LAYER $L_i$ $(i = 1 \cdots m)$

# FIG. 6

# FIG. 7

# FIG. 8

# FIG. 9

PERMISSIBLE RANGE
OF IRRADIATION
SPOT POSITION

RANDOM ERROR

Pm

y

TARGET
POSITION P

PERMISSIBLE RANGE
OF IRRADIATION
SPOT POSITION

x

SHIFT OF SPOT FROM
TARGET POSITION P OF
MEAN POSITION Pm BY
SYSTEMATIC ERROR

# FIG. 10

PERMISSIBLE RANGE Ar
OF RANDOM ERROR

PERMISSIBLE RANGE As
OF SYSTEMATIC ERROR

RANDOM ERROR Er

y

Pm

TARGET
POSITION P

x

SYSTEMATIC ERROR Es

# FIG. 11

# FIG. 12

STEP S5

INPUT THE SET NUMBER OF BEAM IRRADIATION SECTIONS IN ONE SPOT — S6F

IS THE SET NUMBER OF BEAM IRRADIATION SECTIONS 2 OR MORE? — S6G
No / Yes

LAST BEAM IRRADIATION SECTION? — S6H
No / Yes

START DOSE MEASUREMENT IN BEAM IRRADIATION SECTION $S_{i,k}$ — S6I

IS DOSE $Rs_{i,k}$ OF BEAM IRRADIATION SECTION S TARGET DOSE Rs0? — S6J
No / Yes

CLEAR SECOND DOSE MONITOR — S6K

INPUT ACTUAL IRRADIATION POSITION $Pa_{i,j}$ OF IRRADIATION SPOT — S6A

CALCULATE DEVIATION $D_j$ BETWEEN TARGET POSITION $P_{i,j}$ OF IRRADIATION SPOT AND ACTUAL IRRADIATION POSITION $Pa_{i,j}$ — S6B

CALCULATE SYSTEMATIC ERROR $Es_{i,j}$ AND RANDOM ERROR $Er_{i,j}$ — S6C

INPUT ACTUAL IRRADIATION POSITION $Pas_{i,k}$ OF IRRADIATION SPOT IN BEAM IRRADIATION SECTION $S_{i,k}$ — S6L

CALCULATE DEVIATION $d_k$ BETWEEN TARGET POSITION $P_{i,j}$ OF IRRADIATION SPOT AND ACTUAL IRRADIATION POSITION $Pas_{i,k}$ — S6M

CALCULATE SYSTEMATIC ERROR $Ess_{i,k}$ AND RANDOM ERROR $Ers_{i,k}$ — S6N

IS SYSTEMATIC ERROR $Es_{i,j}$ WITHIN FIRST PERMISSIBLE RANGE? — S6D
No / Yes

IS RANDOM ERROR $Er_{i,j}$ WITHIN SECOND PERMISSIBLE RANGE? — S6E
No / Yes

IS SYSTEMATIC ERROR $Ess_{i,k}$ WITHIN FIRST PERMISSIBLE RANGE? — S6O
No / Yes

IS RANDOM ERROR $Ers_{i,k}$ WITHIN SECOND PERMISSIBLE RANGE? — S6P
No / Yes

OUTPUT BEAM IRRADIATION STOP SIGNAL — S18

STEP S7

STOP ION BEAM IRRADIATION — S19

# FIG. 13

FIG. 14

EP 2 910 279 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10118204 A
- JP 2004358237 A
- JP 2011177374 A
- JP 2011206495 A
- US 2012161030 A1